# EUROPÄISCHE PATENTSCHRIFT

(11) **EP 1 646 649 B1**
(45) Veröffentlichungstag und Bekanntmachung des Hinweises auf die Patenterteilung: **27.02.2013**
(21) Anmeldenummer: 04741141.8
(22) Anmeldetag: 19.07.2004
(51) Int. Cl.: C07K 14/47, G01N 33/574, C07K 16/18, C12Q 1/68, A61K 38/17, A61K 39/395, A61P 35/00

(54) **MACC1-POLYPEPTID UND DESSEN VERWENDUNG FÜR DIE TUMORDIAGNOSTIK UND TUMORTHERAPIE**
MACC1-POLYPEPTIDE AND USE THEREOF FOR THE DIAGNOSTIC AND THERAPY OF TUMORS
POLYPEPTIDE MACC1 ET SON UTILISATION DANS LE DIAGNOSTIC TUMORAL ET LA THERAPIE TUMORALE

(30) Priorität: 18.07.2003 DE 10332854
(43) Veröffentlichungstag der Anmeldung: 19.04.2006
(73) Patentinhaber: Charité - Universitätsmedizin Berlin, 10117 Berlin (DE)
(72) Erfinder: STEIN, Ulrike, 16341 Schwanebeck (DE); SCHWABE, Holger, 10243 Berlin (DE); WALTHER, Wolfgang, 16341 Schwanebeck (DE); SCHLAG, Peter, Michael, 13467 Berlin (DE)
(74) Vertreter: Krauss, Jan
(86) Internationale Anmeldenummer: PCT/EP2004/008053
(87) Internationale Veröffentlichungsnummer: WO 2005/010042

(56) Entgegenhaltungen:
- DATABASE EMBL 28. April 2003 (2003-04-28), INTERNATIONAL HUMAN GENOME SEQUENCING CONSORTIUM: "The DNA sequence of Homo spaiens: "similar to expressed sequence AI594717 [Homo sapiens]" XP002305044 Database accession no. XM_294213
- DATABASE Geneseq [Online] 25. Februar 2003 (2003-02-25), "Human liver single exon probe, SEQ ID No 20133." XP002305045 gefunden im EBI accession no. GSN:ABS45143 Database accession no. ABS45143 & WO 01/57273 A (CHEN WENSHENG ; HANZEL DAVID K (US); PENN SHARRON G (US); RANK DAVID R) 9. August 2001 (2001-08-09)
- DATABASE Geneseq [Online] 2. August 2002 (2002-08-02), "Human colon cancer related nucleotide sequence SEQ ID NO:2340." XP002305046 gefunden im EBI accession no. GSN:ABQ58645 Database accession no. ABQ58645 & WO 02/29086 A (BURGESS CHRISTOPHER ; CARROLL EDDIE III (US); CATINO THEODORE J (US);) 11. April 2002 (2002-04-11)
- OTSUKA M ET AL: "Differential expression of the L-plastin gene in human colorectal cancer progression and metastasis" BIOCHEMICAL AND BIOPHYSICAL RESEARCH COMMUNICATIONS, ACADEMIC PRESS, SAN DIEGO, CA, US, Bd. 289, 2001, Seiten 876-881, XP002237813 ISSN: 0006-291X
- BRETT DAVID ET AL: "A rapid bioinformatic method identifies novel genes with direct clinical relevance to colon cancer" ONCOGENE, Bd. 20, Nr. 33, 27. Juli 2001 (2001-07-27), Seiten 4581-4585, XP002305305 ISSN: 0950-9232
- KNOESEL THOMAS ET AL: "Incidence of chromosomal imbalances in advanced colorectal carcinomas and their metastases" VIRCHOWS ARCHIV, Bd. 440, Nr. 2, Februar 2002 (2002-02), Seiten 187-194, XP002305505 ISSN: 0945-6317
- DATABASE UniProt [Online] 1. Oktober 2003 (2003-10-01), SCHWABE ET AL.: "Putative binding protein 7a5." XP002305047 gefunden im EBI accession no. UNIPROT:Q7Z5A5 Database accession no. Q7Z5A5

## Beschreibung

Die vorliegende Erfindung betrifft die Nukleinsäuresequenz, die für das Polypeptid von MACC1 kodiert und dessen Verwendungen, insbesondere für die Tumordiagnostik und Tumortherapie bei metastasierenden Tumoren.

### Beschreibung

Die Entstehung von Metastasen ist ein komplexer mehrstufiger Prozeß, der eine Vielzahl molekularer und zellulärer Veränderungen umfaßt, und prinzipiell in vier definierte Phasen eingeteilt werden kann: a) Wachstum und Vaskularisierung des Primärtumors, b) Ablösen und Eindringen einzelner invasiver Zellen in das Gefäßsystem, c) Dissemination im Gefäßsystem und Extravasation am Zielorgan, und schließlich d) Bildung von Makrometastasen aus den eingewanderten Tumorzellen.

So verlieren bestimmte Zellen eines Primärtumors den Zell-Zell-Kontakt, invadieren durch die extrazelluläre Matrix und werden über Blut- und Lymphsystem verbreitet. Diese Tumorzellen können schließlich extravadieren, sich an bestimmte Zielorgane anheften und dort proliferieren, was zur Ausbildung von Metastasen führt.

Eine Reihe von generell Metastasierungs-assoziierten Molekülen wurden bereits beim Vergleich von Primärtumor und Metastase identifiziert. So führt die Fehlregulation von Zelladhäsionsmolekülen (z.B. Cadherine) in Primärtumoren zum Verlust des Zell-Zell-Kontakts und ermöglicht den mobilisierten Tumorzellen die Invasion und das Eindringen in Blut- und Lymphgefäßsystem. Tumorzellen, die durch Blut- und Lymphgefäßsystem im Körper verteilt wurden, heften sich an die Endothelzellen der Gefäße, vermittelt durch Adhäsionsmoleküle auf den Endothelzellen (z.B. E-Selectin, LP AM, VCAM-1, LuECAM-1, ICAM-1), an die bestimmte Oberflächenmoleküle der Tumorzellen (z.B. VLA-4, LFA-1) selektiv binden können. Im weiteren Verlauf der Metastasierung muß eine Bindung der metastasierenden Tumorzellen an die Komponenten der extrazellulären Matrix erfolgen, z.B. Fibronectin und Vitronectin. Dies wird durch verschiedene Integrine vermittelt, Tumorzellen mit unterschiedlichem Metastasierungspotential unterscheiden sich in der Adhäsion zu den verschiedenen Elementen der extrazellulären Matrix. Von großer Bedeutung für die Extravasation und die Migration der Tumorzellen sind weiterhin die Matrix-degradierenden Enzyme, z.B. die Matrix-Metalloproteinasen (MMPs, insbesondere MMP-9 und MMP-2) im System mit ihren Gewebe-Inhibitoren (Tissue Inhibitors of MMPs, TIMPs). Die Aktivität dieser Moleküle kann über das System Urokinase-Typ-Plasminogen-Aktivator/Urokinase-Typ-Plasminogen-Aktivator-Rezeptor (uPA/uPA-Rezeptor) gewebespezifisch reguliert werden, gezeigt z.B. für die Expression von MMP-9 bei Lebermetastasen kolorektaler Karzinome.

Für die Adhäsion von metastasierenden Tumorzellen an die Zielorgane sind wiederum spezifische Adhäsionsmoleküle (VCAM-1, LPAM, E-Selectin, VLAs, 1CAM-1 und verschiedene Integrine) entscheidend [siehe unter anderem Streit M, et al. Adhesion receptors in malignant transformation and dissemination of gastrointestinal tumors. Recent Results Cancer Res. 1996;142:19-50. Imai K, et al. Regulation of integrin function in the metastasis of colorectal cancer Nippon Geka Gakkai Zasshi. 1998 Jul;99(7):415-8. Krause T, Turner GA. Are selectins involved in metastasis? Clin Exp Metastasis. 1999 May;17(3):183-92, und Portera CA Jr, et al. Molecular determinants of colon cancer metastasis. Surg Oncol. 1998 Nov-Dec;7(3-4):183-95.]. An den Metastasierungszielorganen müssen die Tumorzellen eine bestimmte Mikro-Umgebung vorfinden, um adhärieren und proliferieren zu können ("seed and soil-Hypothese). Tumorzellen können sich zunächst im gesamten Körper verteilen, aber dann nur in bestimmten Organen Metastasen generieren. Der entscheidende Faktor der Metastasierung ist demnach nicht die Migration der Tumorzellen zu den Zielorganen, sondern das Potential der Tumorzellen, in einer bestimmten Umgebung im Zielorgan zu proliferieren. "Schlafende" Tumorzellen können oft jahrelang im Körper vorhanden sein, ohne daß Metastasen nachweisbar sind.

Die oben beschriebene "seed and soil-Hypothese" ist eine der am meisten akzeptierten Theorien für die organspezifische Metastasierung. Daneben existiert eine zweite Theorie, nach der Endothelzellen in den Blutgefäßen der Metastasierungs-Zielorgane bestimmte Adhäsionsmoleküle exprimieren, wodurch zirkulierende Tumorzellen dort festgehalten werden und somit die Metastasierung in diesen spezifischen Organen stattfindet. Die dritte, sogenannte Chemoattraktions-Theorie besagt, daß organspezifische Moleküle in den Blutkreislauf gelangen, die die zirkulierenden Tumorzellen dazu anregt, zu den entsprechenden Metastasierungsorganen zu wandern und dort Metastasen auszubilden.

Die bisherigen Untersuchungen zur organspezifischen Metastasierung machen deutlich, daß offensichtlich in einem längeren Prozeß Gene selektiv angeschaltet (oder abgeschaltet) werden müssen, die zum Zeitpunkt der Verbreitung der Tumorzellen im Körper noch nicht aktiv (bzw. noch aktiv) sind. Daher sind Genexpressions-Analysen unabdingbar, die untersuchen, welche Gene in Metastasen bestimmter Zielorgane im Vergleich zu den entsprechenden Primärtumoren verschieden exprimiert werden.

Jährlich werden ca. 20000 Neuerkrankungen für das Kolonkarzinom in der Bundesrepublik registriert. Für das Kolonkarzinom sind verschiedene Metastasierungsorte bekannt, z.B. Leber, Lymphknoten, Lunge, Knochen und Hirn.

Die Robert-Rössle-Klinik ist eine onkologisch orientierte Klinik mit dem Schwerpunkt Chirurgie. Pro Jahr werden ca. 300 Patienten mit Kolonkarzinomen behandelt, davon weisen ca. 150-170 Patienten Metastasen des Primärtumors auf. Die beobachtete Metastasierungsfrequenz der Zielorgane entspricht der in der Literatur beschriebenen, mit 80% Lebermetastasen und 15% Lungenmetastasen. Die 5-Jahres-Überlebensrate beträgt ca. 20-25%, bei solitären Metastasen (Leber, Lunge) allerdings nur etwa 5%.

Die Leber stellt das bedeutsamste Zielorgan für die Metastasierung des Kolonkarzinoms dar, da die Tumorzellen - vermittelt über die Pfortader - in der Leber festgehalten werden und sich danach zu anderen Organen ausbreiten.

Jedoch ist auch bekannt, daß oftmals Metastasen z.B. in der Lunge oder im Knochen vorhanden sind, ohne daß Metastasen in der Leber festgestellt wurden. Damit stellt das metastasierende Kolonkarzinom ein interessantes Modell für die Identifizierung und Analyse von Genen dar, um eine differentielle Genexpression bei der organspezifischen Metastasierung weiter zu untersuchen.

Die von derartigen Genen kodierten Proteine könnten eine direkte Funktion für die Adhäsion von Tumorzellen an bestimmte Gewebe und Organe haben (organspezifische Adhäsionsmoleküle). Ebenso könnten sie durch Wechselwirkungen mit den normalen Zellen der Metastader Metastasierungsorgane die Zellproliferation und das Wachstum der Metastasen in einer bestimmten Umgebung ermöglichen (z.B. spezifische Rezeptoren und Effektoren). Weiterhin ist denkbar, daß solche Proteine für die intrazelluläre Vorbereitung der Tumorzellen auf die Metastasierung in einem bestimmten Zielorgan benötigt werden, so innerhalb verschiedener Signaltransduklionskaskaden und Regulationsmechanismen. In diesem Zusammenhang sind Proteine mit entsprechenden Protein-Protein-Interaktionsdomänen von Bedeutung, z.B. mit Src-Homology-Domains (SH3-, SH2-Domänen) oder Eps15-Homology-Domains (EH-Domänen). Diese Domänen sind definierte Sequenzmotive, die eine spezifische Bindung an Liganden ermöglichen. SH3-Domänen sind u.a. in solchen Proteinen vorhanden, die bestimmte Liganden zu Kinasen oder deren Substraten geleiten und daher eine wichtige Rolle bei der Regulation von Tyrosinkinase-Signaltransduktionswegen spielen.

Insbesondere ist die Identifizierung solcher Markergene wertvoll, anhand deren Expression im primären Karzinom eine wahrscheinliche Metastasierung in bestimmte Zielorgane vor dem eigentlichen Metastasierungsereignis prognostiziert und, basierend darauf, evtl. verhindert werden kann.

Es ist somit eine Aufgabe der vorliegenden Erfindung, solche weiteren Markergene zur Verfügung zu stellen, mit denen eine verbesserte Diagnose und Therapie im Hinblick auf Metastasierung in bestimmte Zielorgane erreichen läßt.

Diese Aufgabe wird durch die Erfindung, wie in den Patentansprüchen beansprucht, gelöst.

Gemäß einem ersten Aspekt der vorliegenden Erfindung wird diese Aufgabe durch das zur Verfügung stellen einer Nukleinsäuresequenz gelöst, die für das Polypeptid von MACC1 kodiert, ausgewählt aus der Gruppe: a) einer Nukleinsäuresequenz mit der in SEQ ID No: 1 angegebenen Sequenz, b) Nukleinsäuresequenzen, die sich als Ergebnis des degenerierten genetischen Codes von der in SEQ ID No: 1 angegebenen Nukleinsäuresequenz ableiten. Ein weiterer Aspekt betrifft das ebenfalls zur Verfügung gestellte MACC1-Polypeptid mit der in SEQ ID No: 2 angegebenen Aminosäuresequenz, kodiert durch eine erfindungsgemäße Nukleinsäuresequenz.

Zur Aufreinigung der erfindungsgemäßen Polypeptide kann ein weiteres/weiterer Polypeptid("tag") angefügt sein. Protein-tags erlauben beispielsweise die hochaffine Absorption an eine Matrix, stringentes Waschen mit geeigneten Puffern, ohne den Komplex in nennenswertem Maße zu eluieren und anschließend gezielte Elution des absorbierten Komplexes. Beispiele der dem Fachmann bekannten Protein-tags sind ein (His)₆-tag, ein V5-tag, ein Myctag, ein FLAG-tag, ein Strep-tag, ein Strep-tag II, ein Hämagglutinin-tag, Glutathion-Transferase (GST)-tag, Intein mit einem Affinitäts-Chitin-binding-tag oder Maltosebindendes Protein (MBP)-tag. Diese Protein-tags können sich N-, C-terminal und/oder intern befinden.

Neben den aus Zellen isolierten natürlichen Polypeptiden können alle erfindungsgemäßen Polypeptide oder deren Teile unter zellfreien Bedingungen hergestellt worden sein, z. B. durch Synthese oder durch in vitro-Translation. So kann das gesamte Polypeptid oder Teile davon zum Beispiel mit Hilfe der klassischen Synthese (Merrifield-Technik) synthetisiert werden. Teile der erfindungsgemäßen Polypeptide eignen sich insbesondere zur Gewinnung von Antiseren, mit deren Hilfe geeignete Genexpressionsbanken durchsucht werden können, um so zu weiteren funktionellen Varianten des erfmdungsgemäßen Polypeptids zu gelangen.

Neben den aus Zellen isolierten natürlichen Nukleinsäuren können alle erfmdungsgemäßen Nukleinsäuren oder deren Teile auch synthetisch hergestellt worden sein. Weiterhin kann zur Durchführung der Erfindung eine Nukleinsäure verwendet werden, die synthetisch hergestellt worden ist. So kann die erfindungsgemäße Nukleinsäure beispielsweise chemisch anhand der in den von SEQ ID No. 2 beschriebenen Proteinsequenzen unter Heranziehen des genetischen Codes z. B. nach der Phosphotriester-Methode synthetisiert werden (siehe z. B. Uhlmann & Peyman 1990, Chemical Reviews 90:543-584).

Die Zugangsnummer XP_294213.1 (eingetragen am 28-APR-2003 in die Datenbank) beschreibt ein menschliches Polypeptid mit einer Länge von 816 Aminosäuren. Diese als ähnlich zu dem EST AI594717 [*Homo sapiens*] beschriebene Sequenz entspricht in den letzten 813 Aminosäuren der SEQ ID No. 2 und wurde aus NCBI contig NT_007819 durch eine automatisierte Computeranalyse mittels des Programms "GenomeScan" erzeugt. Das Gen ist auf Chromosom 7 lokalisiert. Es sind weder Funktion und noch prognostischer Wert für dieses Gen bekannt. Die vorliegende Erfindung stellt somit die Identifizierung der genomischen DNA-Sequenz, der Vollängen-cDNA und die der putativen Proteinsequenz für MACCI zur Verfügung **(siehe auch Datenbank-Eintrag HGNC:30215 auf der Website des HUGO Gene Nomenclature Committee (HGNC)).**

Ein weiterer Aspekt der Erfindung betrifft ein Oligonukleotid, das spezifisch an eine Nukleinsäuresequenz gemäß der vorliegenden Erfindung hybridisiert, **mit der Nukleinsäuresequenz der SEQ ID NO. 7.** Oligonukleotide stellen wichtige Werkzeuge dar, die zum einen in PCR-Reaktionen, aber auch als Sonden in Hybridisierungsreaktionen verwendet werden können. Weitere Verwendungsmöglichkeiten sind als Therapeutika in der z.B. Gentherapie oder in Mutagenesetechniken vorhanden. Die erfindungsgemäßen Oligonukleotide können in Form von Nukleinsäuren umfassend DNA, dsDNA, RNA, mRNA, siRNA, PNA und/oder CNA vorliegen. Die Oligonukleotide können weiterhin als "sense"- oder "antisense"-Oligonukleotide vorliegen. Für auf Hybridisierung basierende Nachweistechniken können die Oligos darüber hinaus geeignet markiert sein, zum Beispiel durch Farbstoffe, Radionuklide, Enzyme oder Massen- oder Ladungsmarker. Die Markierung richtet sich nach dem jeweiligen Nachweisverfahren, das angewendet werden soll. Die erfindungsgemäßen Oigonukleotide sind vorzugsweise eine DNA, insbesondere eine doppelsträngige DNA mit einer Länge von mindestens 8 Nukleotiden, vorzugsweise mit mindestens 12 Nukleotiden, insbesondere mit mindestens 24 Nukleotiden. Die Obergrenze für Oligonukleotide wird durch die jeweilige praktische Verwendung bestimmt, wobei üblicherweise maximale Länge von 50-200 Nukleotiden bevorzugt sind.

Oligonukleotide werden in der Regel schnell durch Endo- oder Exonukleasen, insbesondere durch in der Zelle vorkommende DNasen und RNasen, abgebaut. Deshalb ist es vorteilhaft, die Nukleinsäure zu modifizieren, um sie gegen den Abbau zu stabilisieren, so daß über einen langen Zeitraum eine hohe Konzentration der Nukleinsäure in der Zelle beibehalten wird (Beigelman et al. 1995, Nucleic Acids Res. 23:3989-94; Dudycz 1995, WO 95/11910; Macadam et al. 1998, WO 98/37240; Reese et al. 1997, WO 97/29116). Typischerweise kann eine solche Stabilisierung durch die Einführung von einer oder mehrerer Intemukleotid-Phosphatgruppen oder durch die Einführung einer oder mehrerer Nicht-Phosphor-Intemukleotide, erhalten werden.

Geeignete modifizierte Intemukleotide sind in Uhlmann und Peymann (1990, Chem. Rev. 90:544) zusammengefaßt (siehe auch Beigelman et al. 1995, Nucleic Acids Res. 23:3989-94; Dudycz 1995, WO 95/11910; Macadam et al. 1998, WO 98/37240; Reese et al. 1997, WO 97/29116). Modifizierte Internukleotid-Phosphatreste und/oder nicht-Phosphoresterbindungen in einer Nukleinsäure, die bei einer der erfindungsgemäßen Verwendungen eingesetzt werden können, enthalten zum Beispiel Methylphosphonat, Phosphorothioat, Phosphoramidat, Phosphorodithioat, Phosphatester, während Nicht-Phosphor-Internukleotid-Analoge, beispielsweise Siloxanbrücken, Carbonatbrücken, Carboxymethylester, Acetamidatbrücken und/oder Thiobrücken enthalten. Es ist auch beabsichtigt, daß diese Modifizierung die Haltbarkeit einer pharmazeutischen Zusammensetzung, die bei einer der erfindungsgemäßen Verwendungen eingesetzt werden kann, verbessert.

Für die Expression des betreffenden Gens ist im allgemeinen eine doppelsträngige DNA bevorzugt, wobei der für das Polypeptid kodierende DNA-Bereich besonders bevorzugt ist. Dieser Bereich beginnt mit dem ersten in einer Kozak-Konsensus-Sequenz (Kozak 1987, Nucleic. Acids Res. 15:8125-48) liegenden Start-Codon (ATG) bis zum nächsten Stop-Codon (TAG, TGA bzw. TAA), das im gleichen Leseraster zum ATG liegt. Eine weitere Verwendung der erfindungsgemäßen Nukleinsäuresequenzen ist die Konstruktion von anti-sense Oligonukleotiden (Zheng und Kemeny 1995, Clin. Exp. Immunol. 100:380-382; Nellen und Lichtenstein 1993, Trends Biochem. Sci. 18:419-23) und/oder Ribozymen (Amarzguioui et al. 1998, Cell. Mol. Life Sci. 54:1175-202; Vaish, et al. 1998, Nucleic Acids Res. 26:5237-42; Persidis 1997, Nat. Biotechnol. 15:921-2; Couture und Stinchcomb 1996, Trends Genet. 12:510-5). Mit "antisense"-Oligonukleotiden kann man die Stabilität der erfindungsgemäßen Nukleinsäure verringern und/oder die Translation der erfindungsgemäßen Nukleinsäure inhibieren. So kann beispielsweise die Expression der entsprechenden Gene in Zellen sowohl *in vivo* als auch *in vitro* verringert werden. Oligonukleotide können sich daher als Therapeutikum eignen. Für die Verwendung als Sonde oder als "antisense" Oligonukleotid ist eine einzelsträngige DNA oder RNA bevorzugt. Die erfindungsgemäßen Oligonukleotide können in Form von Nukleinsäuren umfassend DNA, dsDNA, RNA, mRNA, siRNA, PNA und/oder CNA vorliegen. Ein weiterer besonders bevorzugter Aspekt der vorliegenden Erfindung betrifft Oligonukleotide als Therapeutikum in der Form als siRNA. Diese Art der Gentherapie ist dem Fachmann auch für die Tumortherapie bekannt und kann z.B. aus der folgenden Literatur und den weiteren Referenzen darin entnommen werden. So betreffen Ait-Si-Ali S, Guasconi V, Harel-Bellan A. RNA interference and its possible use in cancer therapy Bull Cancer. 2004 Jan;91(1):15-8; Caplen NJ, Mousses S. Short interfering RNA (siRNA)-mediated RNA interference (RNAi) in human cells. Ann N Y Acad Sci. 2003 Dec;1002:56-62; Caplen NJ. RNAi as a gene therapy approach. Expert Opin Biol Ther. 2003 Jul;3(4):575-86; Lu PY, Xie FY, Woodle MC. siRNA-mediated antitumorigenesis for drug target validation and therapeutics. Curr Opin Mol Ther. 2003 Jun;5(3):225-34; und Oshiumi H, Begum NA, Matsumoto M, Seya T. RNA interference for mammalian cells Nippon Yakurigaku Zasshi. 2002 Aug;120(2):91-5 den allgemeinen technologischen Hintergrund, Devroe E, Silver PA. Therapeutic potential of retroviral RNAi vectors. Expert Opin Biol Ther. 2004 Mar;4(3):319-27. Devroe E, Silver PA. Retrovirus-delivered siRNA. BMC Biotechnol. 2002 Aug 28;2(1):15. Tran N, Cairns MJ, Dawes IW, Arndt GM. Expressing functional siRNAs in mammalian cells using convergent transcription. BMC Biotechnol. 2003 Nov 06;3(1):21. Futami T, Miyagishi M, Seki M, Taira K. Induction of apoptosis in HeLa cells with siRNA expression vector targeted against bcl-2. Nucleic Acids Res Suppl. 2002;(2):251-2. Scherr M, Battmer K, Ganser A, Eder M. Modulation of gene expression by lentiviral-mediated delivery of small interfering RNA. Cell Cycle. 2003 May-Jun;2(3):251-7. Shen C, Reske SN. Adenovirus-delivered siRNA. Methods Mol Biol. 2004;252:523-32. Salmons B, Gunzburg WH. Targeting of retroviral vectors for gene therapy. Hum Gene Ther. 1993 Apr;4(2):129-41, und Kobayashi N, Matsui Y, Kawase A, Hirata K, Miyagishi M, Taira K, Nishikawa M, Takakura Y. Vector-based in vivo RNA interference: dose- and time-dependent suppression of transgene expression. J Pharmacol Exp Ther. 2004 Feb;308(2):688-93. Epub 2003 Nov 10 betreffen bestimmte Vektoren zur siRNA Therapie. Zuletzt beschreiben Naito Y, Yamada T, Ui-Tei K, Morishita S, Saigo K. siDirect: highly effective, target-specific siRNA design software for mammalian RNA interference. Nucleic Acids Res. 2004 Jul 1;32(Web Server issue):W124-9 beispielhaft ein Programm zum Design und dem Aufbau von siRNA-Molekülen.

Ein weiterer Aspekt der vorliegenden Erfindung betrifft das erfindungsgemäße Nukleinsäuremolekül, das erfindungsgemäße Polypeptid oder das erfindungsgemäße Oligonukleotid zur Behandlung von Erkrankungen. Die Möglichkeit der Verwendung dieser Substanzen im Zusammenhang mit menschlichen Erkrankungen war bisher nicht bekannt.

Ein weiterer Aspekt der vorliegenden Erfindung betrifft einen Vektor, vorzugsweise in Form eines Plasmids, shuttle Vektors, Phagemids, Cosmids, Expressionsvektors, adenoviralen Vektors, retroviralen Vektors (Miller, et al. "Improved retroviral vectors for gene transfer and expression", BioTechniques Vol. 7, No. 9, p 980, 1989) und/oder gentherapeutisch wirksamen Vektors, der eine erfindungsgemäße Nukleinsäure enthält. So kann die erfindungsgemäße Nukleinsäure in einem Vektor, vorzugsweise in einem Expressionsvektor oder gentherapeutisch wirksamen Vektor, enthalten sein. Vorzugsweise enthält der gentherapeutisch wirksame Vektor zellspezifische regulatorische Sequenzen, die funktionell mit der erfindungsgemäßen Nukleinsäure verbunden sind. Die Expressionsvektoren können prokaryontische oder eukaryontische Expressionsvektoren sein. Beispiele für prokaryontische Expressionsvektoren sind für die Expression in *E. coli* z.B. die Vektoren pGEM oder pUC-Derivate und für eukaryontische Expressionsvektoren für die Expression in *Saccharomyces cerevisiae* z. B. die Vektoren p426Met25 oder p426GAL1 (Mumberg et al. 1994, Nucleic. Acids Res. 22:5767-5768), für die Expression in Insektenzellen z. B. *Baculovirus-*Vektoren wie in EP-B1-0 127 839 oder EP-B1-0 549 721 offenbart, und für die Expression in Säugerzellen z. B. die Vektoren Rc/CMV und Rc/RSV oder SV40-Vektoren, welche alle allgemein erhältlich sind.

Im allgemeinen enthalten die Expressionsvektoren auch für die jeweilige Wirtszelle geeignete Promotoren, wie z. B. den trp-Promotor für die Expression in *E. coli* (siehe z. B. EP-B1-0 154 133), den Met 25, GAL 1 oder ADH2-Promotor für die Expression in Hefen (Russel et al. 1983, J. Biol. Chem. 258:2674-2682), den Baculovirus-Polyhedrin-Promotor, für die Expression in Insektenzellen (siehe z. 13. EP-B1-0 127 839). Für die Expression in Säugetierzellen sind beispielsweise Promotoren geeignet, die eine konstitutive, regulierbare, gewebsspezifische, zellzyklusspezifische oder metabolischspezifische Expression in eukaryontischen Zellen erlauben. Regulierbare Elemente gemäß der vorliegenden Erfindung sind Promotoren, Aktivatorsequenzen, Enhancer, Silencer und/oder Repressorsequenzen. Beispiel für geeignete regulierbare Elemente, die konstitutive Expression in Eukaryonten ermöglichen, sind Promotoren, die von der RNA Polymerase II erkannt werden oder virale Promotoren, CMV-Enhancer, CMV-Promotor, SV40-Promotor oder LTR-Promotoren z. B. von MMTV (mouse mammary tumour virus; Lee et al. 1981, Nature 214:228-232) und weitere virale Promotor- und Aktivatorsequenzen, abgeleitet aus beispielsweise HBV, HCV, HSV, HPV, EBV, HTLV oder HIV. Beispiele für regulierbare Elemente, die regulierbare Expression in Eukaryonten ermöglichen, sind der Tetracyclinoperator in Kombination mit einem entsprechenden Repressor (Gossen et al. 1994, Curr. Opin. Biotechnol. 5:516-20).

Um die Einführung von erfindungsgemäßen Nukleinsäuren und damit die Expression des Polypeptids in einer eu- oder prokaryontischen Zelle durch Transfektion, Transformation oder Infektion zu ermöglichen, kann die Nukleinsäure als Plasmid, als Teil eines viralen oder nicht-viralen Vektors vorliegen. Als virale Vektoren eignen sich hierbei besonders: Retroviren, Baculoviren, Vakziniaviren, Adenoviren, adenoassoziierte Viren und Herpesviren. Als nicht-virale Vektoren eignen sich hierbei besonders: Virosomen, Liposomen, kationische Lipide, oder poly-Lysin konjugierte DNA.

Beispiele von gentherapeutisch wirksamen Vektoren sind Virusvektoren, beispielsweise Adenovirusvektoren oder retrovirale Vektoren (Lindemann et al., 1997, Mol. Med. 3:466-76; Springer et al. 1998, Mol. Cell. 2:549-58).

Ein bevorzugter Mechanismus, erfindungsgemäße Polypeptide *in vivo* zur Expression zu bringen, ist der virale Gen-Transfer, insbesondere mit Hilfe retroviraler Partikel. Diese werden vorzugsweise genutzt, entsprechende Zielzellen, vorzugsweise T-Lymphozyten, des Patienten *ex vivo* mit den für erfindungsgemäße Polypeptide kodierenden Genen oder Nukleotidsequenzen durch Transduktion zu versehen. Die Zielzellen können daraufhin im Sinne eines adoptiven Zelltransfers wieder in den Patienten reinfundiert werden, um mit der *de novo* eingefügten Spezifität tumorizide und/oder immunmodulierende Effektorfunktionen zu übernehmen. Es wurden auf diesem Wege sehr gute gentherapeutische Erfolge in der Behandlung der durch Immuninkompetenz gekennzeichneten SCID-X1- Krankheit bei Neugeborenen erzielt, indem hämatologische Vorläuferzellen mit einem analogen intakten Transgen einer in den Kindern vorkommenden nicht-funktionellen mutierten Variante des χ-Kettengens, das für die Differenzierung in die verschiedenen Effektorzellen des adaptiven Immunsystems essentiell ist, retroviral versehen wurden (Cavazzana-Calvo et al., 2000).

Weiterhin besteht die Möglichkeit, den Gentransfer *in vivo* durchzuführen, einerseits durch präferentiell stereotaktische Injektion der infektiösen Partikel, andererseits durch direkte Applikation von Viren-produzierenden Zellen (Oldfield, et al. Hum. Gen. Ther., 1993, 4:39-69).

Die zum Transfer von Genen häufig eingesetzten viralen Vektoren sind nach heutigem Stand der Technik vorwiegend retrovirale, lentivirale, adenovirale und adeno-assoziierte virale Vektoren. Diese sind von natürlichen Viren abgeleitete zirkuläre Nukleotidsequenzen, in denen zumindest die viralen Strukturprotein-kodierenden Gene durch das zu transferierende Konstrukt ausgetauscht werden. Neue, nicht-virale Vektoren bestehen aus autonomen, sich selbstintegrierenden DNS-Sequenzen, den Transposonen, die durch z.B. liposomale Transfektion in die Wirtszelle eingeschleust werden und erstmals erfolgreich zur Expression humaner Transgene in Säugerzellen eingesetzt wurden (Yant et al., 2000).

Gentherapeutisch wirksame Vektoren lassen sich auch dadurch erhalten, daß man die erfindungsgemäße Nukleinsäure mit Liposomen komplexiert, da damit eine sehr hohe Transfektionseffizienz, insbesondere von Hautzellen, erreicht werden kann (Alexander und Akhurst 1995, Hum. Mol. Genet. 4:2279-85). Bei der Lipofektion werden kleine unilamellare Vesikel aus kationischen Lipiden durch Ultraschallbehandlung der Liposomensuspension hergestellt. Die DNA wird ionisch auf der Oberfläche der Liposomen gebunden, und zwar in einem solchen Verhältnis, daß eine positive Nettoladung verbleibt und die Plasmid-DNA zu 100% von den Liposomen komplexiert wird. Neben den von Felgner et al. (1987, supra) eingesetzten Lipidmischungen DOTMA (1,2-Dioleyloxpropyl-3-trimethylammoniumbromid) und DPOE (Dioleoylphosphatidylethanolamin) wurden inzwischen zahlreiche neue Lipidformulierungen synthetisiert und auf ihre Effizienz der Transfektion verschiedener Zellinien getestet (Behr et al. 1989, Proc. Natl. Acad. Sci. USA 86:6982-6986; Felgner et al. 1994, J. Biol. Chem. 269:2550-25561; Gao und Huang. 1991, Biochim. Biophys. Acta 1189:195-203). Beispiele der neuen Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin). Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. 1999, Gene Therapy 6:282; Brandén et al 1999, Nature Biotech. 17:784). Hilfsstoffe umfassen auch Moleküle, die die Freisetzung von Nukleinsäuren in das Cytoplasma der Zelle ermöglichen (Kiehler et al 1997, Bioconj. Chem. 8:213) oder beispielsweise Liposomen (Uhlmann und Peymann 1990, supra). Eine andere besonders geeignete Form von gentherapeutischen Vektoren läßt sich dadurch erhalten, daß man die erfindungsgemäße Nukleinsäure auf Goldpartikeln aufbringt und diese mit Hilfe der sogenannten "Gene Gun" in Gewebe, bevorzugt in die Haut, oder Zellen schießt (Wang et al., 1999, J. Invest. Dermatol. 112:775-81). Ein weiterer Aspekt betrifft die Einbringung "nackter DNA" mit der Gene gun, wie zum Beispiel in T Niidome und L Huang; Gene Therapy Progress and Prospects: Nonviral vectors; Gene Therapy (2002) 9, 1647-1652 und den Referenzen darin beschrieben.

Für die gentherapeutische Anwendung der erfindungsgemäßen Nukleinsäure ist es auch von Vorteil, wenn der Teil der Nukleinsäure, der für das Polypeptid kodiert, ein oder mehrere nicht kodierende Sequenzen einschließlich Intronsequenzen, vorzugsweise zwischen Promotor und dem Startcodon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Jackson 1993, Cell 74:9-14 und Palmiter et al. 1991, Proc. Natl. Acad. Sci. USA 88:478-482).

Ein weiterer Aspekt der vorliegenden Erfindung betrifft dann einen rekombinanten prokaryontischer oder eukaryontischer Wirtsorganismus, der mindestens eine erfindungsgemäße Nukleinsäuresequenz oder mindestens einen erfindungsgemäßen Vektor enthält. Der Wirtsorganismus kann aus einer diploiden Zelle, einer Pflanzenzelle, einer Säugerzelle, einer Nematodenzelle, einer Fischzelle, einer Insektenzelle und, insbesondere, einer nicht-menschlichen Stammzelle bestehen. Ein bevorzugtes Beispiel wäre eine Maus-Stammzelle. Weiterhin stellt die Erfindung einen rekombinanten nicht-menschlichen Organismus zur Verfügung, insbesondere einen genetisch defizienten oder "Knock-out"-Säuger (wie eine Ziege oder Schaf), -Nager (wie ein Kaninchen, Maus, Ratte oder Hamster), - Nematode (wie Caenorhabditis elegans), -Fisch (wie ein Zebrafisch), -Pflanze (wie Arabidopsis thaliana, Mais, Reis, Weizen oder Kartoffel), -Insekt oder Qualle, bei dem das entsprechende Gen für 7a5/Prognostin mutiert oder deletiert wurde. Diese Versuchsorganismen sind wertvolle Hilfsmittel und können z.B. zum Screening nach Bindungspartnern verwendet werden (siehe z.B. unten). Verfahren zur Herstellung solcher Organismen sind dem Fachmann aus der einschlägigen Literatur bekannt.

Ein weiterer Gegenstand der vorliegenden Erfindung ist ein polyklonaler oder monoklonaler Antikörper oder ein Antigen-bindendes Fragment davon zur Diagnose, Prognose und Therapie-Optimierung von mit MACC1 Expression assoziierten Erkrankungen oder zur Identifizierung von pharmakologisch aktiven Substanzen, der gegen ein erfindungsgemäßes MACC1 Polypeptid gerichtet ist und mit den erfindungsgemäßen MACC1- Polypeptiden spezifisch reagiert, wobei die oben genannten Teile des Polypeptids entweder selbst immunogen sind oder durch Kopplung an geeignete Träger, wie z. B. bovines Serumalbumin, immunogen gemacht bzw. in ihrer Immunogenität gesteigert werden können. Dieser Antikörper ist entweder polyklonal oder monoklonal, bevorzugt ist ein monoklonaler Antikörper. Unter dem Begriff Antikörper versteht man gemäß der vorliegenden Erfindung auch gentechnisch hergestellte und gegebenenfalls modifizierte Antikörper bzw. antigenbindende Teile davon, wie z.B. chimäre Antikörper, humanisierte Antikörper, multifunktionelle Antikörper, bi- oder oligospezifische Antikörper, einzelsträngige Antikörper, F(ab)- oder F(ab)₂-Fragmente (siehe z.B. EP-B1-0 368 684, US 4,816,567, US 4,816,397, WO 88/01649, WO 93/06213, WO 98/24884). Die erfindungsgemäßen Antikörper können zur Diagnose, Therapie-Überwachung und/oder Behandlung von mit MACC1 Expression assoziierten Erkrankungen oder zur Identifizierung von pharmakologisch aktiven Substanzen verwendet werden. Ein weiterer Aspekt betrifft die Herstellung von Antikörpern (pAK, mAK) für die Evaluierung histologischer Schnitte.

Ein weiterer Aspekt der Erfindung betrifft ein Verfahren zur Herstellung eines Antikörpers, vorzugsweise eines polyklonalen oder monoklonalen Antikörpers zur Diagnose und/oder Behandlung von mit MACC1 Expression Erkrankungen oder zur Identifizierung von pharmakologisch aktiven Substanzen, dadurch gekennzeichnet, daß ein Antikörper produzierender Organismus mit einem erfindungsgemäßen Polypeptid oder funktioneller Äquivalente davon oder Teile davon mit mindestens 6 Aminosäuren, vorzugsweise mit mindestens 8 Aminosäuren, insbesondere mit mindestens 12 Aminosäuren oder einer erfindungsgemäßen Nukleinsäure immunisiert wird.

Das Verfahren erfolgt nach dem Fachmann allgemein bekannten Methoden durch Immunisieren eines Säugetiers, beispielsweise eines Kaninchens, mit dem erfindungsgemäßen Polypeptid oder den genannten Teilen davon oder diese(s) kodierende Nukleinsäure(n), gegebenenfalls in Anwesenheit von z. B. inkompletten Freundsches Adjuvant und/oder Aluminiumhydroxidgelen (siehe z. B. Diamond et al. 1981, The New England Journal of Medicine, pp. 1344-1349). Die im Tier aufgrund einer immunologischen Reaktion entstandenen polyklonalen Antikörper lassen sich anschließend nach allgemein bekannten Methoden leicht aus dem Blut isolieren und z. B. über Säulenchromatographie reinigen. Monoklonale Antikörper können beispielsweise nach der bekannten Methode von Winter & Milstein (1991, Nature 349: 293-299) hergestellt werden.

Ein noch weiterer Aspekt der vorliegenden Erfindung betrifft dann eine pharmazeutische Zusammensetzung, umfassend ein erfindungsgemäßes Nukleinsäuremolekül ein erfindungsgemäßes Polypeptid, ein erfindungsgemäßes Oligonukleotid oder einen erfindungsgemäßen Antikörper, gegebenenfalls zusammen mit einen pharmazeutisch akzeptablen Träger und/oder Hilfsstoff. figer pharmazeutisch-technologischer Verfahren. Hierzu werden die erfindungsgemäßen Nukleinsäuremoleküle, erfindungsgemäßes Polypeptid, erfindungsgemäße Oligonukleotide oder ein erfindungsgemäßer Antikörper mit geeigneten, pharmazeutisch annehmbaren Hilfs- und Trägerstoffen zu den für die verschiedenen Indikationen und Applikationsorten geeigneten Arzneiformen verarbeitet.

Dabei können die Arzneimittel in der Weise hergestellt werden, daß die jeweils erwünschte Freisetzungsrate, z.B. eine rasche Anflutung und/oder ein Retard- bzw. Depoteffekt erzielt werden. Ein Medikament kann dabei eine Salbe, Gel, Pflaster, Emulsion, Lotion, Schaum, Creme oder mischphasigen oder amphiphile Emulsionssysteme (Öl/Wasser-Wasser/Öl-Mischphase), Liposom, Transfersom, Paste oder Puder sein.

Der Begriff "Hilfsstoff' bedeutet erfindungsgemäß jedes, nicht-toxische, feste oder flüssige Füll-, Verdünnungs-, oder Verpackungsmaterial, solange es nicht ungebührend nachteilhaft mit einem erfindungsgemäßen Nukleinsäuremolekül, einem erfindungsgemäßen Polypeptid, einem erfindungsgemäßen Oligonukleotid oder einem erfindungsgemäßen Antikörper oder dem Patienten reagiert. Flüssige galenische Hilfsstoffe sind zum Beispiel steriles Wasser, physiologische Kochsalzlösung, Zuckerlösungen, Ethanol und/oder Öle. Galenische Hilfsstoffe zur Herstellung von Tabletten und Kapseln können zum Beispiel Bindemittel und Füllmaterial enthalten.

Weiterhin kann ein erfindungsgemäßes Nukleinsäuremolekül, ein erfindungsgemäßes Polypeptid, ein erfindungsgemäßes Oligonukleotid und/oder ein erfindungsgemäßer Antikörper in Form von systemisch eingesetzten Arzneimitteln verwendet werden. Dazu gehören die Parenteralia, zu denen die Injektabilia und Infusionen gehören. Injektabilia werden entweder in Form von Ampullen oder auch als sog. gebrauchsfertige Injektabilia, z.B. als Fertigspritzen oder Einmalspritzen, daneben auch in Durchstechflaschen zur mehrmaligen Entnahme hergerichtet. Die Verabreichung der Injektabilia kann in Form der subkutanen (s.c.), intramuskulären (i.m.), intravenösen (i.v.) oder intrakutanen (i.c.) Applikation erfolgen. Insbesondere können die jeweils zweckmäßigen Injektionsformen als Kristallsuspensionen, Lösungen, nanopartikuläre oder kolloiddisperse Systeme, wie z.B. Hydrosole, hergestellt werden.

Die injizierbaren Zubereitungen können ferner als Konzentrate hergestellt werden, die mit wässrigen isotonischen Verdünnungsmitteln aufgelöst oder dispergiert werden. Die Infusionen lassen sich ebenfalls in Form von isotonischen Lösungen, Fettemulsionen, Liposomenzubereitungen, Mikroemulsionen, zubereiten. Wie Injektabilia können auch Infusionszubereitungen in Form von Konzentraten zum Verdünnen zubereitet werden. Die injizierbaren Zubereitungen können auch in Form von Dauerinfusionen sowohl in der stationären als auch in der ambulanten Therapie, z.B. in Form von Minipumpen, appliziert werden.

Das erfindungsgemäße Nukleinsäuremolekül, erfindungsgemäße Polypeptid, erfindungsgemäße Oligonukleotid oder ein erfindungsgemäßer Antikörper kann in den Parenteralia an Microcarrier oder Nanopartikel gebunden sein, beispielsweise an feinstverteilte Partikel auf Basis von Poly(meth)acrylaten, Polylactaten, Polyglycolaten, Polyaminsäuren oder Polyetherurethanen. Die parenteralen Zubereitungen können auch als Depotpräparate modifiziert sein, z.B. aufbauend auf dem "Multiple Unit Prinzip", wenn ein erfindungsgemäßes Nukleinsäuremolekül, ein erfindungsgemäßes Polypeptid, ein erfmdungsgemäßes Oligonukleotid oder ein erfindungsgemäßer Antikörper in feinst verteilter bzw. dispergierte, suspendierter Form oder als Kristallsuspension eingearbeitet ist oder aufbauend auf dem "Single Unit Prinzip", wenn ein erfindungsgemäßes Nukleinsäuremolekül, ein erfindungsgemäßes Polypeptid, ein erfindungsgemäßes Oligonukleotid oder ein erfindungsgemäßer Antikörper eingeschlossen ist in einer Arzneiform, z.B. ein Tablette oder ein Stäbchen, das anschließend implantiert wird. Häufig bestehen diese Implantate oder Depotarzneimittel bei Single Unit- und Multiple Unit-Arzneiformen aus sogenannten bioabbaubaren Polymeren, wie z.B. Polyester der Milch- und Glykolsäure, Polyetherurethanen, Polyaminosäuren, Poly(meth)acrylaten oder Polysacchariden.

Als Hilfs- und Trägerstoffe bei der Herstellung von Parenteralia kommen Aqua sterilisata, den pH-Wert beeinflussende Substanzen, wie z.B. organische und anorganische Säuren und Basen sowie deren Salze, Puffersubstanzen zur Einstellung des pH-Wertes, Isotonisierungsmittel, wie z.B. Natriumchlorid, Natriumhydrogencarbonat, Glucose und Fructose, Tenside bzw. oberflächenaktive Substanzen und Emulgatoren, wie z.B. Partialfettsäureester des Polyoxyethylensorbitans (Tween®) oder z.B. Fettsäureester des Polyoxyethylens (Cremophor®), fette Öle, wie z.B. Erdnussöl, Sojabohnenöl und Rizinusöl, synthetische Fettsäureester, wie z.B. Ethyloleat, Isopropylmyristat und Neutralöl (Miglyol®), sowie polymere Hilfsstoffe sie z.B. Gelatine, Dextran, Polyvinylpyrrolidon, von die Löslichkeit erhöhenden Zusätzen organischer Lösungsmittel wie z.B. Propylenglycol, Ethanol, N,N-Dimethylacetamid, Propylenglycol oder komplexbildender Stoffe, wie z.B. Citraten und Harnstoff, Konservierungsmittel, wie z.B. Benzoesäurehydroxypropyl- und -methylester, Benzylalkohol, Antioxidantien, wie z.B. Natriumsulfit und Stabilisatoren, wie z.B. EDTA, in Betracht.

Bei Suspensionen erfolgt ein Zusatz von Verdickungsmitteln zum Verhindern des Absetzens von erfindungsgemäßen Nukleinsäuremolekülen, erfindungsgemäßen Polypeptiden, erfindungsgemäßen Oligonukleotiden oder einem erfindungsgemäßen Antikörper von Tensiden und Peptisatoren, um die Aufschüttelbarkeit des Sediments zu sichern, oder von Komplexbildnern wie EDTA. Es lassen sich auch mit verschiedenen Polymeren Wirkstoffkomplexe erzielen, beispielweise mit Polyethylenglykolen, Polystyrol, Carboxymethylcellulose, Pluronics® oder Polyethylenglykolsorbitfettsäureestern. Zur Herstellung von Lyophilisaten werden Gerüstbildner, wie z.B. Mannit, Dextran, Saccharose, Humanalbumin, Lactose, PVP oder Gelatinesorten verwendet.

Die jeweils geeigneten Arzneiformen lassen sich in Einklang mit dem Fachmann bekannten Rezepturvorschriften und Verfahrensweisen auf der Basis pharmazeutisch-physikalischer Grundlagen herstellen.

Ein letzter Aspekt betrifft die Verabreichung der erfindungsgemäßen Nukleinsäuren und/oder Oligonukleotide bei der Gentherapie mittels herkömmlicher Transfektionssystem, wie zum Beispiel Liposomen oder "Particle gun"-Techniken oder "Gene gun mit "nackter DNA", wie oben bereits beschrieben.

Ein weiterer Gegenstand der vorliegenden Erfindung ist eine Wirtszelle, die mit einem erfindungsgemäßen Vektor oder einem anderen erfindungsgemäßen Genkonstrukt transfiziert ist. Wirtszellen können sowohl prokaryontische als auch eukaryontische Zellen sein, Beispiele für prokaryontische Wirtszellen sind *E*. *coli* und für eukaryontische Zellen *Saccharomyces cerevisiae* oder Insektenzellen. Weitere brauchbare Zellen und Organismen sind bereits oben beschrieben.

Ein weiterer Aspekt der vorliegenden Erfindung betrifft dann eine diagnostische Zusammensetzung, umfassend eine erfindungsgemäße Nukleinsäuresequenz, ein erfindungsgemäßes Polypeptid, ein erfindungsgemäßes Oligonukleotid oder einen erfindungsgemäßen Antikörper. Die erfindungsgemäße diagnostische Zusammensetzung kann auch als Bestandteil eines diagnostischen Kits der Erfindung vorliegen (siehe unten).

Ein noch weiterer Aspekt der vorliegenden Erfindung betrifft dann ein Verfahren zur Diagnose von tumoralen Erkrankungen, **wobei die tumorale Erkrankung Kolonkrebs ist,** umfassend den Schritt von Bestimmen der Expression von **MACC1** in einer biologischen Probe aus einem erkrankten Gewebe und Vergleich der Expression mit der Expression von **MACC1** in einem gesunden Gewebe, **wobei MACC1 ein erfindungsgemäßes MACC1-Polypeptid ist**. Bevorzugt ist das erfindungsgemäße Verfahren, bei dem die Bestimmung der Expression von **MACC1** eine Hybridisierung, eine PCR, eine "real time" (RT)-PCR, eine Antigen-Antikörper Bindung, einen ELISA, eine optische Proteom-Analyse, eine ein- oder mehrdimensionale Gelelektrophorese, eine massenspektrometrische Analyse, eine Chromatographie, eine Sequenzierung, Methylierungsanalyse, SNP-Bestimmung oder Kombinationen dieser Verfahren umfaßt.

So stellt die vorliegende Erfindung die Etablierung, und Aufwendung des transkriptionellen Expressionsnachweises von **MACC1** unter Verwendung u.a. der real time RT-PCR zur Verfügung. Beispielhaft wurde die Expressionshöhe von **MACC1** in der unbekannten Probe (z.B. Tumor-RNA) als Prozent der **MACC1-Expression** in der kommerziell verfügbaren, humanen Kolonkarzinom-Kalibrator-Zellinie SW620 ermittelt.

Ein weiterer Aspekt der vorliegenden Erfindung betrifft dann ein Verfahren zur Diagnose von tumorösen Erkrankungen, wobei die tumoröse Erkrankung metastasierender Kolonkrebs ist. Aus dem oben genannten ergibt sich ein prognostischer Wert der auf den oben genannten Wegen generierten **MACC1-Expressionshöhen** als klinische Parameter. Es gibt bisher keine Beschreibung von Korrelationen der Expressionen der **MACC1**-Transkripte mit klinischen Parametern wie Metastasierung und/oder Metastasen-freies bzw. generelles Überleben. So führte in in vitro-Systemen die Transfektion der **MACC1**-cDNA zu erhöhtem Wachstum und zu gesteigerter Migration von humanen Kolonkarzinomzellen. In in in vivo-Experimenten wurden sowohl im subkutanen als auch im orthotopen Tiermodell erhöhte Wachstumcharakteristika der transfizierten Zellklone gemessen. Im orthotopen Modell kam es darüber hinaus zur Bildung von Metastasen in der Leber. Die Korrelation der Expressionen der MACC1-Transkripte insbesondere mit der Prognose für die Fernmetastasierung der Kolonkarzinome war bisher unbekannt und ist überraschend.

Erfindungsgemäß kann eine zu untersuchende biologische Probe aus einer Tumorbiopsie aus Darm, Leber, Lymphknoten, Lunge, Knochen oder Hirn abgeleitet sein. Es sind aber auch andere biologische Proben, wie Speichel, Urin, Blut oder Teile davon möglich.

Die vorliegende Erfindung beschreibt weiterhin ein Verfahren zur Behandlung von tumorösen Erkrankungen, umfassend eine Modulation der Expression von MACC1. Die Erfindung beschreibt die Korrelation der (u.a. transkriptionellen) Expressionshöhe von MACC1 mit der Organspezifität der Metastasierung, sowie der Fernmetastasierungswahrscheinlichkeit von u.a. Kolonkarzinomen. Damit ist die potentielle Nutzung dieses neuen Gens als Markergen für Tumordiagnostik und, darüber hinaus, als Interventionstarget auch für Tumortherapie zur Beeinflussung (Verhinderung) der Fernmetastasierung beim u.a. Kolonkarzinom gegeben. Erfindungsgemäß wird eine Beeinflussung zur Behandlung von tumorösen Erkrankungen durch die Verabreichung einer erfindungsgemäßen wie oben angegebenen pharmazeutischen Zusammensetzung beschrieben. Die vorliegenden Erfindung beschreibt somit ein Verfahren zur Behandlung von tumorösen Erkrankungen, wobei die tumoröse Erkrankung metastasierender Kolonkrebs ist.

Die Identifizierung der Rolle von MACC1 bei der Metastasierung in tumorösen Erkrankungen stellt in einem anderen Aspekt der vorliegenden Erfindung die Möglichkeit der Verwendung von MACC1 als ein "Target" für ein Verfahren zur Auffindung von Substanzen, die an MACC1 binden, zur Verfügung, wobei MACC1 ein erfindungsgemäßes MACC1-Polypeptid ist. Erfindungsgemäß umfaßt dieses Verfahren ein in Kontakt bringen einer Zelle, die MACC1 exprimiert (zum Beispiel einer rekombinanten Zelle wie oben) mit einer Kandidaten-Substanz, Nachweisen der Anwesenheit der Kandidaten-Substanz, die an MACC1 bindet, und Bestimmen, ob die Kandidaten-Substanz auch an MACC1 bindet. Verfahren zur routinemäßigen Durchführung solcher Screenings sind dem Fachmann im Stand der Technik der Pharmazie gut bekannt. Mittels "High-Throughput-Technologien" können geeignete Substanzbibliotheken durchsucht werden. Diese Bibliotheken und ihre Durchsuchung sind dem Fachmann bekannt und leicht an die Gegebenheiten der vorliegenden Erfindung anzupassen, ohne dabei erfinderisch tätig sein zu müssen.

Ein weiterer Aspekt der vorliegenden Erfindung betrifft dann ein Verfahren zur Herstellung einer pharmazeutischen Zusammensetzung, umfassend die Schritte des oben genannten Screening-Verfahrens und geeignetes Formulieren der in Schritt c) identifizierten Substanz in eine pharmazeutisch akzeptable Form, **wobei die Substanz ein Antikörper gegen MACC1 ist**.

Ein weiterer Aspekt der vorliegenden Erfindung betrifft dann die Verwendung einer erfindungsgemäßen Nukleinsäuresequenz, eines erfindungsgemäßen Polypeptids, eines erfindungsgemäßen Oligonukleotids oder eines erfindungsgemäßen Antikörpers zur Diagnose von tumorösen Erkrankungen, **wobei die tumoröse Erkrankung Kolonkrebs ist**. Dazu sind oben Aspekte bereits ausgeführt. Weiter kann anhand von Untersuchungen der Expressionsveränderungen des **MACC1** eines Patienten auch die Pharmakokinetik des verwendeten Therapeutikums zur Behandlung bestimmt werden, und so die Parameter der Behandlung geeignet modifiziert werden (wobei auch andere bekannte Parameter dem Fachmann bekannt sind). Dieser Aspekt ist ebenfalls als "personalized medicine" bekannt. Des weiteren können auch Linker für die bildgebende Diagnostik oder Therapie an erfindungsgemäße Gegenstände angebracht werden (molekulares Imaging, gerichtete Therapie). Diese Linker sind dem Fachmann bekannt, z.B. aus Trail PA, King HD, Dubowchik GM. Monoclonal antibody drug immunoconjugates for targeted treatment of cancer. Cancer Immunol Immunother. 2003 May;52(5):328-37. Epub 2003 Jan 16.; Signore A, Annovazzi A, Chianelli M, Corsetti F, Van de Wiele C, Watherhouse RN. Peptide radiopharmaceuticals for diagnosis and therapy. Eur J Nucl Med. 2001 Oct;28(10):1555-65. Epub 2001 Jul 31. Erratum in: Eur J Nucl Med 2001 Nov;28(11):1737.; und Mehvar R. Dextrans for targeted and sustained delivery of therapeutic and imaging agents. J Control Release. 2000 Oct 3;69(1):1-25.

**Die vorliegende Erfindung beschreibt weiterhin** die Verwendung einer erfindungsgemäßen Nukleinsäuresequenz als Marker für humane Erbkrankheiten. Dazu wird die Sequenz des **MACC1** auf spezifische Mutationen (z.B. SNPs oder andere Punktmutationen) hin untersucht, die dann unterschiedliche Präferenzen für eine Metastasierung bewirken könnten. Auch kann eine erfindungsgemäße Nukleinsäuresequenz oder erfindungsgemäßes Oligonukleotid zur Gentherapie verwendet werden. Aspekte dazu sind bereits oben ausgeführt und schließen siRNA und/oder antisense-Techniken ein.

Zuletzt umfaßt die vorliegende Erfindung auch einen diagnostischen Kit, der eine diagnostische Zusammensetzung wie oben ausgeführt, gegebenenfalls zusammen mit geeigneten Puffern und/oder Gebrauchsanweisungen umfaßt. In einer Ausführungsform liegt der erfindungsgemäße diagostische Kit in Form eines PCR-, insbesondere RT-PCR-, oder ELISA-Kits vor. Ein Bespiel eines solchen Kits für die Echtzeit ("real time") RT-PCR wäre z.B. mit den durch die Erfinder designten Primers und Sonden für den transkriptionellen Expressionsnachweis von MACC1 zusammen mit allen Agenzien für die real time RT-PCR. Darüber hinaus wurde durch die Erfinder ein reproduzierbares und einfach handhabbares Arbeitsprotokoll erstellt. Ein weiterer Aspekt der Erfindung ist die Einbeziehung von MACC1 (unter Anwendung aller Technik-üblichen Kontrollen) in die DNA-Chip-Technologie; sowohl in DNA-Chips, die, jeweils aktualisiert, "alle" bekannten Gene erfassen, als auch insbesondere in solche, die Themen-relevant spezifische Gene z.B. definierter Tumorentitäten, Signaltransduktionskaskaden etc. beinhalten.

Die Verwendung des Nachweises der Expression des hier beschriebenen, neu identifizierten Gen MACC1 (7a5/Prognostin) im Primärtumor von Kolonkarzinom-Patienten ist unter anderem mit der Fernmetastasierung (z.B. in Leber und Lunge) korreliert. Die Erfinder haben durch vergleichende Expressionsanalyse in humanen Primärtumoren, Metastasen unterschiedlicher Zielorgane und in den korrespondierenden Normalgeweben von Kolonkarzinom-Patienten das neue Gen MACC1(7a5/Prognostin) identifiziert. Es ist auf Chromosom 7 des humanen Genoms lokalisiert.

Die transkriptionelle Expression von MACC1 ist überraschenderweise a) in malignen Geweben (Primärtumoren, Metastasen) höher als in den korrespondierenden Normalgeweben, b) in den Fernmetastasen der Kolonkarzinome, z.B. in Leber und Lunge, höher als im korrespondierenden Primärtumor, c) vor allem höher in den Primärtumoren, die bereits metastasiert haben oder im Verlauf der Erkrankung noch manifest metastasieren werden, im Vergleich zu den Primärtumoren, die nicht metastasieren.

Da die hier vorgestellte Erfindung eines Verfahrens zur Femmetastasierungsprognose für Kolonkarzinom-Patienten basierend auf dem transkriptionellen Nachweis des neuen Gens MACC1 eine zentrale klinische Frage berührt, kann ein Interesse an dieser Erfindung ohne Einschränkung für alle onkologischen Kliniken und Forschungsinstitute weltweit gelten.

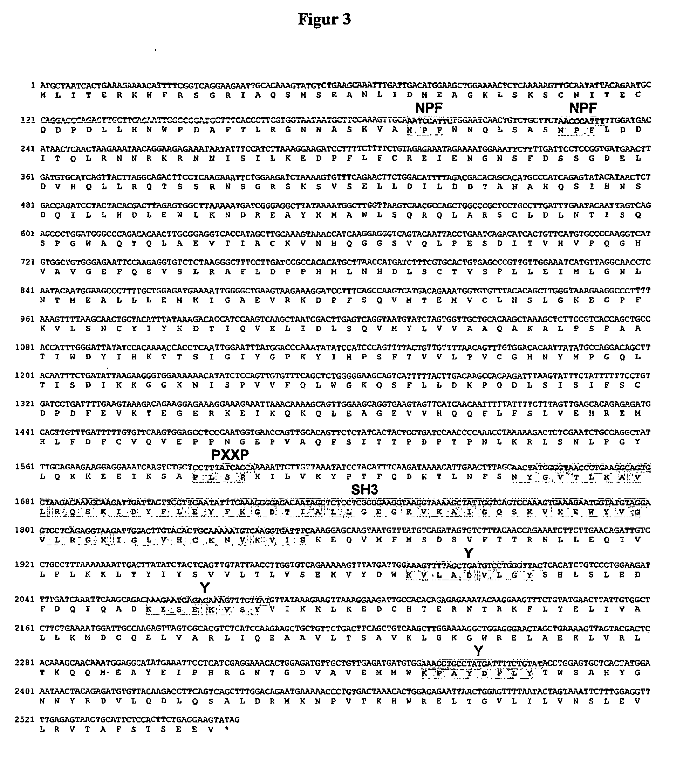

Insbesondere stellt die Erfindung folgende Vorteile zur Verfügung
a) die Sequenz der neu identifizierten MACC1 cDNA sowie der putativen Proteinsequenz, wobei charakteristische Interaktionsdomänen markiert sind (Figur 3).
b) in in vitro-Systemen führte die Transfektion der MACC1 -cDNA zu erhöhtem Wachstum und zu gesteigerter Migration der humanen Kolonkarzinomzellen.
c) in in vivo-Experimenten wurden sowohl im subkutanen als auch im orthotopen Tiermodell erhöhte Wachstumscharakteristika der transfizierten Zellklone gemessen. Im orthotopen Modell kam es darüber hinaus zur Bildung von Metastasen in der Leber.
d) Messung der relativen MACC1-mRNA-Expression in Primärtumoren, Metastasen sowie korrespondierenden Normalgeweben in Patienten mit Kolonkarzinomen.
e) Messung der relativen MACC1-mRNA-Expression in Primärtumoren von Patienten mit Kolonkarzinomen.

Da sich die Verwendung der real time RT-PCR zunehmend zur "state of the art"-Methode der transkriptionellen Expressionsanalytik entwickelt, können die apparativen Voraussetzungen zur Anwendung der Erfindung zunehmend in den Laboratorien weltweit vorausgesetzt werden.

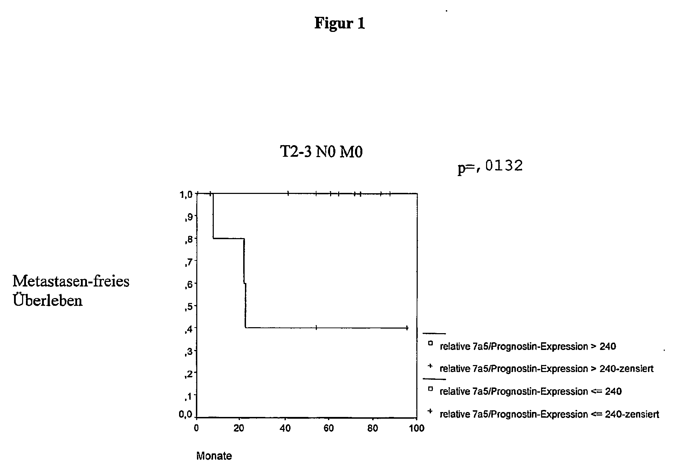

Die Erfindung soll nun im folgenden unter Bezugnahme auf die beigefügten Figuren und Sequenzen weiter beispielhaft erläutert werden, in denen
Figur 1 die relative MACC1-mRNA-Expression in Primärtumoren von Patienten mit Kolonkarzinomen das Metastasen-freies Überleben zeigt,

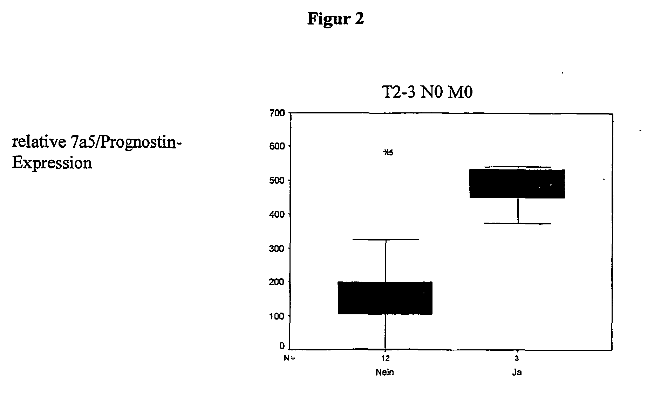

Figur 2 die relative MACC1-mRNA-Expression in Primärtumoren von Patienten mit Kolonkarzinomen die Entwicklung von Metastasen zeigt,
Figur 3 die Sequenz der neu identifizierten MACC1-cDNA sowie der putativen Proteinsequenz zeigt; charakteristische Interaktionsdömanen sind markiert,
Figur 4 die MACC1-Expression in Primärtumoren von Kolonkarzinom-Patienten, gruppiert nach Metastasierung und Metastasierungszeitpunkt, zeigt,
Figur 5 das Metastasen-freies Überleben vom Kolonkarzinom-Patienten mit niedriger (≤240) und hoher (>240) MACC1-Expression in Primärtumoren zeigt, und
Figur 6 den inhibitorischen Einfluß von MACC1-spezifischer siRNA auf die Expression von 7a5/Prognostin (A) und den inhibitorischen Einfluß von MACC1-spezifischer siRNA auf das Migrationsverhalten von Kolonkarzinomzellen (B) zeigt,
SEQ ID No. 1: die cDNASequenz von MACC1 zeigt,
   SEQ ID No. 2: die Proteinsequenz von MACC1 zeigt,
   SEQ ID No. 3 bis SEQ ID No. 6: in den Beispielen verwendete Oligonukleotide, und
   SEQ ID No. 7: ein beispielhaftes siRNA-Molekül aus den Beispielen zeigt.

### Beispiele

### Metastasierungsprognose unter Verwendung des MACC1-mRNA-Expressionsniveaus (real time RT-PCR)

### Patienten und menschliche Gewebe

71 Patienten traten in diese Studie ein, die mit informierter Zustimmung der Patienten durchgeführt wurde. Die Mensch-primären Kolontumore (46 Proben von 44 Patienten), Metastasen (52 Proben von 41 Patienten) und normalen Geweben (37 Mucosaproben, 18 Lebergewebe, 1 Lungengewebe, und 1 Lymphknoten; von 40 Patienten) wurden in flüssigem Stickstoff perioperativ schockgefroren und bei -80°C für weitere Analysen in der Tumor Bank der Robert-Rössle-Klinik, Berlin gelagert. Der TNM Status wurde durch Pathologen der Robert-Rössle- Klinik bestimmt.

### Mikrodissektion und RNA Isolierung

Serielle Kryoschnitte jedes Gewebes wurden für die RNA Isolierung (10µm) und Immunohistochemie (5µm) hergestellt. Für die Mikrodissektion von Tumour-Zellpopulationen, wurde jeder zehnte Kryoschnitt pro Gewebe durch einen Pathologen nach Färbung mit Hämalaun

### Differential Display RT-PCR

Differential Display RT-PCR wurde unter der Verwendung des RNAimage Differential Display Systems 3 (GenHunter Corp., Nashville, TN) durchgeführt. Jeder der drei zur Verfügung gestellten eine Base-verankerten Primer wurde für die reverse Transkription von 200 ng von Gesamt-RNA pro Reaktion verwendet, gemäß den Anweisungen des Herstellers. Differential display PCR wurde unter Befolgung des empfohlenen Protokolls durchgeführt, wobei α-[³³P]dATP (NEN Life Science, Zaventum, Belgium) als Marker und AmpliTaq Polymerase (Applied Biosystems, Weiterstadt, DE) verwendet wurden. Die PCR-Produkte wurden in einem 5% Polyacrylamidgel getrennt. Das Gel wurde geblottet, getrocknet und über Nacht gegenüber Röntgenfilm ausgesetzt (Kodak BioMax MR, via Sigma, Deisenhofen, DE). Der Film wurde wieder an das Gel angepaßt, um ein Ausschneiden der Banden zu ermöglichen, die verschiedene Expression zeigten. Diese cDNA-Fragmente wurden durch PCR unter der Verwendung derselben Primerkombinationen und PCR Bedingungen wie für differential Display RT-PCR reamplifiziert, und in den pCR2.1 Vektor (was zu den hier beschriebenen Konstrukten führte: pCR2.1/7a3, pCR2.1/7a5, pCR2.1/7a10, Original TA Cloning Kit, Invitrogen) kloniert.

### Überprüfung von cDNA Fragment-enthaltenden Konstrukten

Die pCR2.1 Konstrukte (hier beschrieben: pCR2.1/7a3, pCR2.1/7a5, pCR2.1/7a10) wurden unter der Verwendung eines Minifold II Slotblot Apparates (Schleicher & Schuell, Dassel, DE) jedes auf fünf Nylonmembranen unter der Verwendung eines Minifold II Slotblot Apparates (Schleicher & Schuell, Dassel, DE) geblottet. Jede Membran wurde mit vollständigen differential Display RT-PCR Produkten, die aus einem spezifischen Gewebetyp erzeugt wurden (Consalez et al. 1996), hybridisiert. Um eine hohe spezifische Aktivität zu erreichen, wurden die differential Display RT-PCR Produkte mit α-[³²P]dCTP (NEN) neu markiert (Random Primed Labeling Kit, Roche Diagnostics), und wurden gereinigt (Nucleotide Removal Kit, QIAGEN, Hilden, DE). Die Hybridisierung wurde über Nacht bei 65°C (6x SSC, 5x Denhardt's Reagenz, 0.5% SDS, 100µg/ml gescherter DNA) durchgeführt. Die Blots wurden gegenüber Röntgenfilm ausgesetzt (Kodak Xomat-AR). Die Inserts von Konstrukten die ein ähnliches Expressionsmuster zeigten, wie es in der differential Display RT-PCR gefunden wurde, wurden sequenziert (Invitek, Berlin, DE).

### cDNA Bibliotheks-Screening

Die SW480 Zellinien-abgeleitete menschliche kolorektale Adenocarcinom STRETCH PLUS cDNA library (Clontech, Heidelberg, DE) wurde in den bakteriellen Wirtsstamm Y1090 r⁻ transfiziert. Zweifache Filter-Replika wurden unter der Verwendung von Optitran verstärkte Cellulosenitrat Membranen (Schleicher & Schuell) erzeugt. Die Filter wurden denaturiert (0.5N NaOH, 1M NaCl) und neutralisiert (0.5M Tris pH 8, 1M NaCl). Das Konstrukt pCR2.1/7a5 wurde mit α-[³²P]dCTP (NEN) unter der Verwendung des Random Primed Labeling Kit (Roche Diagnostics) markiert als Sonde verwendet. Die Hybridisierung wurde über Nacht bei 65°C (5x SSC, 5x Denhardt's Reagenz, 0.1% SDS, 100µg/ml gescherte DNA) durchgeführt. Die Filter wurden dann gegenüber Röntgenfilm exponiert (Kodak Xomat-AR). Positive Plaques wurden gepickt. Die DNA Präparation wurde unter der Verwendung des Lambda-DNA Präparations Kit (QIAGEN) durchgeführt. Die cDNA Inserts wurden sequenziert (Invitek).

### Verlängerung der Sequenzinformation

Durch differential Display RT-PCR und cDNA library Screening erhaltene Sequenzen wurden für die Datenbankanalysen verwendet, die alle unter der Verwendung des WWW²HUSAR Analyse Pakets auf dem Server der Biocomputing Service Gruppe des DKFZ in Heidelberg (http://genius.embnet.dkfz-heidelberg.de). Ein EST Cluster wurde bestimmt, der Sequenzidentität zeigte, lokalisiert auf dem genomischen DNA Sequenzfragment AC007001. In der 5'-Richtung wurden drei weitere EST Cluster lokalisiert. EST-verbindende RT-PCR wurde durchgeführt (GeneAmp RNA PCR Kit, Applied Biosystems) um zu überprüfen, ob die identifizierten EST Cluster Teil der 7a5-cDNA sind. Unter der Verwendung der neuen Sequenzinformation und der cDNA Sequenz von SH3BP4, die Sequenzähnlichkeiten in überlappenden Regionen zeigte, wurden EST Cluster identifiziert, die auf dem genomischen DNA Sequenzfragment AC005083 lokalisiert waren. Long-distance RT-PCR (reverse Transkription: Expand Reverse Transkriptase; PCR: Expand High Fidelity PCR, beide Roche Diagnostics) wurde durchgeführt um zu überprüfen, ob die identifizierten ESTs Teile der MACC1-cDNA waren.

### 5'-RACE

5'-RACE wurde durchgeführt, wobei der SMART RACE cDNA Amplification Kit (Clontech) gemäß den Anweisungen des Herstellers verwendet wurde. Agarosegel-getrennte PCR Produkte wurden re-amplifiziert und in den pCR2.1 Vektor (Invitrogen) für die Sequenzierung kloniert.

### Klonierung des ORF in einen Expressionsvektor

Long distance RT-PCR wurde wie oben durchgeführt, wobei jedoch Primer für die Amplifikation des gesamten ORF ohne Stopcodon verwendet wurden. Der Primer am 5'-Ende war so aufgebaut, um eine gerichtete Klonierung des ORF in den pcDNA3.1D/V5-His-TOPO Vektor (pcDNA3.1Directional TOPO Expression Kit, Invitrogen), im Hinblick auf den richtigen Rahmen zu ermöglichen. Das Klonierungsverfahren wurde gemäß den Anweisungen des Herstellers durchgeführt. Die Konstrukte (pcDNA3.1D/7a5-V5-His), die das erwartete Insert enthielten, wurden sequenziert.

### Erzeugung von stabil transduzierten MACC1 exprimierenden SW480 Zellklonen

Um die biologische Funktion des MACC1(7a5) Gens zu analysieren, wurden das MACC1 exprimierende pcDNA3.1D/7a5-V5-His Konstrukt und das LacZ-exprimierende pcDNA3.1D/V5-His/lacZ Konstrukt (Invitrogen, diente als Kontrolle) in SW480 Zellen unter der Verwendung von Lipofectin (Invitrogen) gemäß den Anweisungen des Herstellers transduziert. Für die Transduktion, wurden 10µg Plasmid DNA und 15µl Lipofectin verwendet, um 5x 10⁴ Zellen zu transduzieren. 48h nach Transduktion wurden Konstrukt-tragende Klone in G418 (Invitrogen) enthaltendem Medium selektiert. Nach zwei Wochen waren Kolonien sichtbar, die dann für weitere Experimente ring-kloniert und expandiert wurden. Alle isolierten Zellklone wurden durch Western Blots und real time RT-PCR auf MACC1 oder LacZ Expression gescreent.

### Western Blots von MACC1 exprimierenden SW480 Zellklonen

MACC1-transduzierte, lacZ- transduzierte und Wildtyp SW480 Zellen wurden in 1% SDS, 10mM Tris-HCl, 2mM EDTA lysiert und in einem 7.5% Polyacrylamidgel getrennt. Nach Elektroblotting (1 h, Trans Blot - Semi Dry Transfer Cell, BioRad, Munich, DE) wurde exprimiertes MACC1 Protein unter der Verwendung eines anti-V5 Antikörpers (Invitrogen) und einem sekundären anti-Maus-HRP Antikörper (Promega, Madison, WI) nachgewiesen. Die Chemilumineszenz-Reaktion wurde mit ECL Lösung (Amersham Biosciences, Freiburg, DE) gemäß den Anweisungen des Herstellers durchgeführt

### Relative quantitative zwei-Schritt real time RT-PCR

Reverse Transkriptase Reaktion (RT) wurde mit 50 ng an Gesamt-RNA (MuLV Reverse Transkriptase, Applied Biosystems) durchgeführt. Für jede quantitative real time PCR (95°C für 60 Sekunden, 45 Zyklen von 95°C 10 Sekunden, 60°C 10 Sekunden, 72°C 20 Sekunden), wurde 1/5 des RT Volumens unter Verwendung des LightCycler (LightCycler DNA Master Hybridization Probes kit, Roche Diagnostics) genommen. Die Expression von MACC1 und des Haus-keeping-Gens Glucose-6-Phosphat Dehydrogenase (G6PDH) wurden in zweifachen Ansätzen aus der selben RT-Reaktion bestimmt. Für 7a5 wurde ein 136 bp Amplikon und für G6PDH ein 136 bp Amplikon durch Primer hergestellt, die durch Gen-spezifische Fluoreszin - und LCRed640-markiert Hybridisierungssonden (Synthese von Primern und Sonden: BioTeZ und TIB MolBiol, beide Berlin, DE) erkannt wurden. Die Kalibrator cDNA wurde in seriellen Verdünnungen gleichzeitig in jedem Lauf verwendet, abgeleitet von der Zellinie SW620.

Zum Nachweis von menschlichen Zellen SW620 Klontumoren in Mausleber nach orthotoper Transplantation wurde eine menschliche Satelliten Sequenz verwendet. Primer (Synthese BioTeZ, Berlin), die ein Amplikon erzeugten, wurden verwendet (ähnlich zu Becker et al., 2002) (Synthese TIB MolBiol, Berlin). Real time PCR wurde wie oben beschrieben unter der Verwendung von 250 ng der genomischen DNA durchgeführt.
Primer:
   vorwärts: 5' - ttc ttt tga ttc ctc cgg tga - 3' (SEQ ID No. 3)
   reverse: 5' - act ctg atg ggc atg tgc tg - 3' (SEQ ID No. 4)
Sonden (hier für Anwendung am Light Cycler):
   5'- gca gac ttc ctc aag aaa ttc tgg aag atc ta - 3' - FITC (SEQ ID No. 5)
   LCRed - 5'- agt gtt tca gaa ctt ctg gac att tta gac ga - 3'(SEQ ID No. 6)
Annealing-Temperatur: 60°C; Zyklenanzahl: 45

### Soft Agar Wachstum von MACC1 transduzierten SW480 Tumorzellklonen

Zur Evaluierung von Zellwachstum in Soft Agar, wurden 5 ml von auf 50°C vorgewärmtem 1% Agar (Difco, Detroit, MI) mit 5 ml DMEM Medium gemischt (doppelt konzentriert w/o Phenolrot, Invitrogen), ergänzt mit 10% FCS und Antibiotika. 200µl Zellsuspension, die 5x 10⁴ Zellen enthielt wurden schnell zu den 10ml von Soft Agar Gemisch hinzugefügt, kräftig gemischt, in 5ml Aliquots in 60 mm Petrischalen gegossen und für 5 Minuten auf Eis plaziert. Dann wurden die MACC1 transduzierten Zellklone, die lacZ- transduzierten Zellklone und Wildtyp SW480 Zellen wurden in zweifachen Ansätzen in Soft Agar für 8 Tage angezogen und Kolonien wurden gezählt.

### Zellmigrations-Test

Transwell Membrankammern (Porengröße 12,0 µm, Costar, Heidelberg, DE) wurden mit 1ml RPMI 1640 Medium equilibriert, das mit 10% FCS für 24 h bei 37°C und 5% CO₂ ergänzt war. Dann wurde das Medium entfernt und 0,5ml Zellsuspension, die 2,5 x 10⁵ Zellen enthielt wurde zu den Filter-Membrankammern hinzugefügt und 1,5 ml Medium wurden zu der unteren Kammer hinzugefügt. Die Zahl von Zellen, die durch die Membran zu der unteren Kammer wanderten wurde gezählt nach 24 h nach Zellinokulation (in dreifachen Ansätzen durchgeführt).

### In vivo Wachstum von MACC1 transduzierten SW480 Zellklonen: subkutane Transplantation

Für *in vivo* Tumorwachstum wurden 1 x 10⁷ MACC1-transduzierte, LacZ-transduzierte oder Wildtyp SW480 Zellen subkutan entlang der linken Flanke von 6 - 8 Wochen alten männlichen NMRI nu/nu Mäusen an Tag 0 transplantiert. Jede Tiergruppe bestand aus 10 Tieren. Die Tiere wurden für 39 Tage gehalten, um die Entwicklung von meßbaren Tumoren zu ermöglichen. Die Tumorgröße wurde in allen Gruppen in zwei Dimensionen an Tagen 7, 11, 15, 21, 27, 32, 35 und 39 gemessen. Die Tumor-Volumen wurden berechnet (Breite² x Länge)/2 und als mittleres Tumor-Volumen in cm³ angegeben. Die Tiere wurden getötet und Tumore wurden entfernt und in flüssigem Stickstoff für weitere Analysen schockgefroren.

### In vivo Wachstum von MACC1 transduzierten SW480 Zellklonen: orthotope Transplantation

1 x 10⁷ MACC1 transduzierte, LacZ- transduzierte oder Wildtyp SW480 Zellen wurden zuerst subkutan entlang der linken Flanke von 6 - 8 Wochen alten weiblichen SCID-beige Mäusen transplantiert, und für drei Wochen wachsen gelassen. Dann wurden diese Tumore entnommen, in 2 x 1 x 1 mm³ große Stücke geschnitten und wurden orthotop in das Caecum von weiblichen SCID-beigen Mäusen transplantiert. Jede Tiergruppe bestand aus 5 Tieren. Die Tiere wurden für 70 Tage gehalten, um Tumorwachstum und Metastasierung zu ermöglichen. Beginnend am Tag 16, wurde die Tumorgröße in allen Gruppen einmal die Woche gemessen. Die Tiere wurden getötet und Tumore sowie potentiell metastatische Organe wurden entfernt und in flüssigem Stickstoff für weitere Analysen schock-gefroren. Nach der Entnahme wurden die finalen Tumorvolumen Länge x Breite x Höhe berechnet und als mittleres Tumor-Volumen in cm³ angegeben.

### In situ Hybridisierung in menschlichem Geweben

Das MACC1-spezifische differential Display RT-PCR Fragment wurde via pCR2.1/7a5 für Klonierung in den pBluescript II SK+ Vektor (Stratagene GmbH, Heidelberg, DE) übertragen. Die *in vitro* Transkription wurde unter der Verwendung von T7 und SP6 RNA Polymerasen (Epicentre Technologies, Madison, Sonden durchgeführt, WI) um sense und antisense Sonden zu erhalten. Die Transkription wurde unter der Verwendung des DIG Labeling Kit (Roche Diagnostics) durchgeführt.

Kryoschnitte von menschlichem Gewebe wurden in Paraformaldehyd fixiert und getrocknet. Die Prähybridisierung wurde für eine Stunde bei 55°C unter der Verwendung von 4x SSC, 5% Dextransulfat, 1x Denhardt's Reagenz, 50% Formamid, 0,25mg/ml Hefe-tRNA und 0,5mg/ml fragmentierter Lachssperma DNA durchgeführt. Die Hybridisierungsreaktion wurde für 16 Stunden bei 55 °C, unter der Verwendung der Prähybridisierungslösung durchgeführt, die 500 ng/ml der *in vitro*-transkribierten RNA Sonde enthielt. Extensives Waschen wurde in 2x SSC, 50% Formamid bei 55°C durchgeführt. Blocking wurde unter der Verwendung von Blocking Reagenz (Roche Diagnostics) gemäß den Anweisungen des Herstellers durchgeführt. Die Schnitte wurden für zwei Stunden mit Anti-DIG-AP Fab Fragmenten (Roche Diagnostics) inkubiert. Nach Waschen wurde ein kolorimetrischer Nachweis unter der Verwendung von NBT/BCIP Lösung (Roche Diagnostics) durch Inkubation im Dunklen durchgeführt.

### Immunohistochemie

Kryoschnitte von primären Tumoren, Lymphknoten, Lungen- und Leber-Metastasen sowie aus normalen entsprechenden Geweben wurden gemäß einer Modifikation der DAKO Katalysierten Signal Amplifikation (CSA) System (DAKO A/S, Glostrup, Denmark) immungefärbt: Endogene Biotin Reaktivität wurde mittels einer UVC-Bestrahlung bei 254 nm für 35 Minuten bei Raumtemperatur blockiert. Die Schnitte wurden in einer wäßrigen 0,01% NaN₃ Stabilisator-Lösung für 30 Minuten bei 50°C rehydriert. Endogene Peroxidase wurde durch Inkubation mit 3% H₂O₂ in Methanol für 30 Minuten bei 50°C blockiert. Der Protein Block wurde für 5 Minuten wie empfohlen durchgeführt.

Der primäre polyklonale Kaninchen-anti-Mensch MACC1 Antikörper wurde gegen ein MACC1-derived Polypeptid (AA 812 - 826 nahe dem C-Terminus lokalisiert: (KLH)-SALDRMKNPVTKHWR, Eurogentec, Seraing, Belgium) erzeugt und wurde anschließend durch Affinitäts-Chromatographie auf dem Peptidantigen, gekoppelt an CNBr-Sepharose (BioGenes, Berlin, DE), gereinigt. Dieser Antikörper wurde für die Immunohistochemie in einer Verdünnung von 1:1000 verwendet. Um die nicht-spezifische Antikörper-basierte Fc-Rezeptorligation zu vermeiden, wurde ein Fc-depletierter biotinylierter affini-reiner F(ab')₂ Fragment-Antikörper mit anti-Kaninchen Spezifität (Verdünnung 1:40.000, Dianova-Jackson ImmunoResearch, Hamburg, DE) verwendet. Die Streptavidin-HRP wurde für 15 Minuten bei Raumtemperatur angewendet. Kolorimetrischer Nachweis wurde unter der Verwendung von DAB Substrat durchgeführt, wobei dem Protokoll des Hersteller gefolgt wurde. Nuclei wurden mit Hämatoxylin für 1 Minute gegengefärbt. Die Schnitte wurden gemäß topographischer Expressionsaspekte analysiert.

### Statistische Analyse

Das Ausmaß an statistischer Signifikanz wurde unter der Verwendung des nichtparametrischen zwei-seitigen Mann-Whitney Rank Sum Tests (real-time RT-PCR) berechnet, und dem zwei-unabhängige Proben T-Test (Soft Agar Wachstum, Zellmigrationstest, in vivo Wachstum).

### Diagnose auf Basis von MACC1 Expression in Primärtumoren von Kolonkarzinom-Patienten mit und ohne Metastasierung

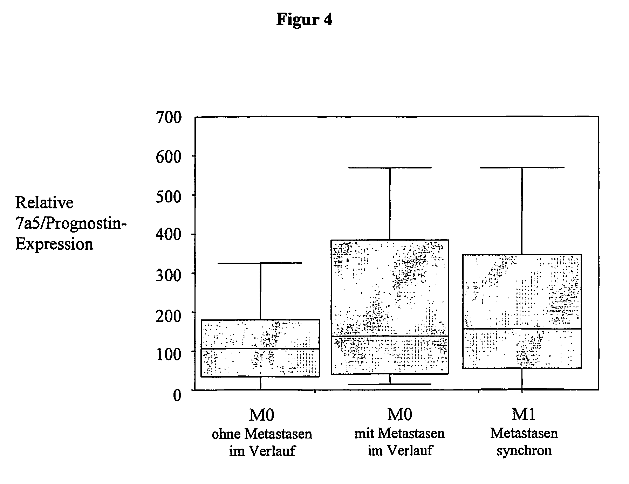

Abbildung 4 (Box plot mit Darstellung der Mediane) zeigt die Höhe der MACC1 Expression in den Primärtumoren von Kolonkarzinom-Patienten (T₂₋₄N₀₋₁),
a) die zum Zeitpunkt der MACC1-Analyse nicht metastasiert waren und im Beobachtungszeitraum von 60 Monaten nicht metastasierten = M0 ohne Metastasen im Verlauf,
b) die zum Zeitpunkt der MACC1-Analyse nicht metastasiert waren, jedoch im Beobachtungszeitraum von 60 Monaten metastasierten = M0 mit Metastasen im Verlauf, und
c) die zum Zeitpunkt der MACC1-Analyse bereits metastasiert waren = M1

In den Primärtumoren der Patienten, die im Verlauf Metastasen entwickelten und die bereits metastasiert waren, wurden dabei deutlich höhere MACC1-Expressionen gemessen.

### Metastasen-freies Überleben von Kolonkarzinom-Patienten in Abhängigkeit von der Höhe der MACC1 Expression

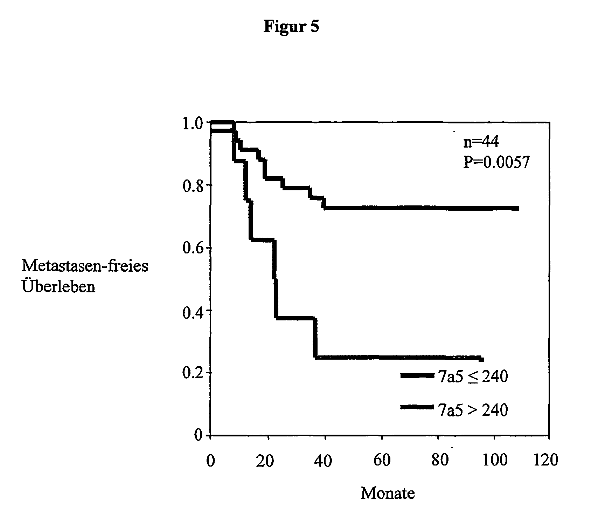

Abbildung 5 (Kaplan-Meier-Kurve) zeigt die Wahrscheinlichkeit für das Metastasen-freie Überleben in Abhängigkeit von niedriger bzw. hoher MACC1-Expression im jeweiligen Primärtumor. Als "cut-off ist der relative MACC1-Wert von 240 kalkuliert, als "niedrig" sind alle relativen MACC1-Werte ≤ 240 definiert und als "hoch" sind alle relativen MACC1-Werte > 240 definiert.
Beispiel 1: Patienten mit MACC1-Expressionen ≤240 im Primärtumor entwickeln zu etwa 20-25% Metastasen (im Beobachtungszeitraum).
Beispiel 2: Patienten mit MACC1-Expressionen >240 im Primärtumor entwickeln zu etwa 75-80% Metastasen (im Beobachtungszeitraum).

Die MACC1-Expression im Primärtumors ist signifikant prädiktiv für die Entwicklung Die MACC1-Expression im Primärtumor ist signifikant prädiktiv für die Entwicklung distanter Metastasen (P=0.0057).

### Therapeutische Intervention mit MACC1-spezifischer siRNA auf Expressionsebene

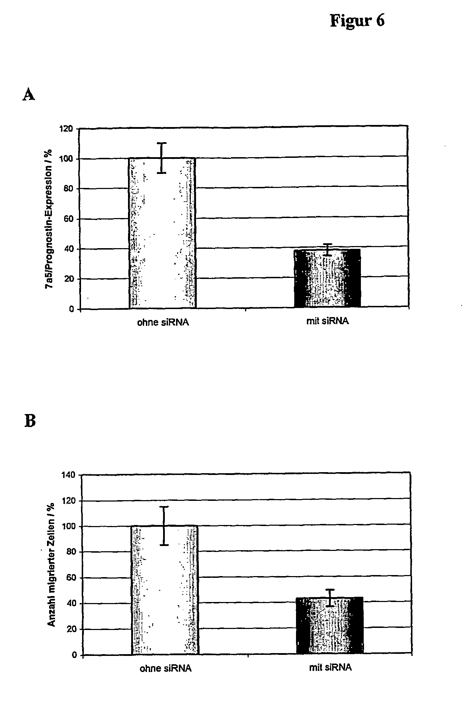

Abbildung 6A beschreibt den Einfluß von transient transfizierten, MACC1 spezifischen siRNA-Oligonukleotiden auf die transkriptionelle Expression von MACC1 in einem stabil MACC1-transfizierten und stark überexprimierenden Zellklon einer humanen Kolonkarzinom-Zelllinie. Die nicht mit siRNA-behandelte Kontrolle entspricht 100%:
Beispiel für ein wirksames MACC1-spezifisches siRNA-Oligonukleotid:
   - nt 2233 bis 2253 der Vollängen cDNA-Sequenz von MACC1
   - 5' -AAGCTTGGAAAAGGCTGGAGG- 3' (SEQ ID No. 7)

### Intervention mit MACC1-spezifischer siRNA auf funktioneller Ebene

Abbildung 6B beschreibt den Einfluß von transient transfizierten MACC1-spezifischen siRNA-Oligonukleotiden auf das Migrationsverhalten eines stabil MACC1-transfizierten und stark überexprimierenden Zellklons einer humanen Kolonkarzinom-Zelllinie. Die nicht mit siRNA-behandelte Kontrolle entspricht 100%. Für das Protokoll des Migrationsassays, siehe oben.

Durch die transiente Transfektion von MACC1-spezifischer siRNA wird die Expression von MACC1 inhibiert. Die Konsequenz besteht in der verminderten Migrationsaktivität dieser Zellen als einem biologischen Parameter für das Metastsierungspotenzial. Für die therapeutische Anwendung am Patienten werden die MACC1 spezifisches siRNA-Oligonukletide bevorzugt in entsprechende Plasmide kloniert. So wird eine stabile siRNA-Expression im Primärtumor des Patienten erreicht, die dann zu einer andauernden Inhibierung der MACC1-Expression führt und die Metastasierung vermindern kann.

## Patentansprüche

1. Nukleinsäuresequenz, die für das Polypeptid von MACC1 kodiert, ausgewählt aus der Gruppe:
a) einer Nukleinsäuresequenz mit der in SEQ ID No: 1 angegebenen Sequenz,
b) Nukleinsäuresequenzen, die sich als Ergebnis des degenerierten genetischen Codes von der in SEQ ID No: 1 angegebenen Nukleinsäuresequenz ableiten.

2. MACC1-Polypeptid mit der in SEQ ID No: 2 angegebenen Aminosäuresequenz, kodiert durch eine Nukleinsäuresequenz nach Anspruch 1.

3. Oligonukleotid, das spezifisch an eine Nukleinsäuresequenz nach Anspruch 1 hybridisiert, mit der Nukleinsäuresequenz der SEQ ID No: 7.

4. Nukleinsäuremolekül nach Anspruch 1, Polypeptid nach Anspruch 2 oder Oligonukleotid nach Anspruch 3 als Arzneimittel.

5. Vektor enthaltend eine Nukleinsäuresequenz nach Anspruch 1.

6. Rekombinanter prokaryontischer oder nicht-menschlicher eukaryontischer Wirtsorganismus enthaltend mindestens einen Vektor gemäß Anspruch 5.

7. Polyklonaler oder monoklonaler Antikörper oder Antigen-bindendes Fragment davon, der ein MACC1-Polypeptid mit SEQ ID No: 2 erkennt.

8. Pharmazeutische Zusammensetzung, umfassend eine Nukleinsäuresequenz nach Anspruch 1, ein Polypeptid nach Anspruch 2, ein Oligonukleotid nach Anspruch 3 oder einen Antikörper nach Anspruch 7, gegebenenfalls zusammen mit einen pharmazeutisch akzeptablen Träger.

9. Diagnostische Zusammensetzung, umfassend eine Nukleinsäuresequenz nach Anspruch 1, ein Polypeptid nach Anspruch 2, ein Oligonukleotid nach Anspruch 3 oder einen Antikörper nach Anspruch 7.

10. Verfahren zur Diagnose von tumoralen Erkrankungen, umfassend den Schritt von Bestimmen der Expression von MACC1 in einer biologischen Probe aus einem erkrankten Gewebe oder Körperflüssigkeiten und Vergleich der Expression mit der Expression von MACC1 in einem gesunden Gewebe oder Körperflüssigkeit, wobei die tumorale Erkrankung Kolonkrebs ist,
wobei MACC1 ein Polypeptid, wie in Anspruch 2 definiert, ist.

11. Verfahren zur Prognose der Wahrscheinlichkeit der Fernmetastasierung von Kolonkrebs, umfassend den Schritt von Bestimmen der Expression von MACC1 in einer biologischen Probe aus einem erkrankten Gewebe oder Körperflüssigkeiten und Vergleich der Expression mit der Expression von MACC1 in einem gesunden Gewebe oder Körperflüssigkeit,
wobei MACC1 ein Polypeptid, wie in Anspruch 2 definiert, ist.

12. Verfahren zur Diagnose von Kolonkrebs nach Anspruch 10 oder zur Prognose der Wahrscheinlichkeit der Fernmetastasierung von Kolonkrebs nach Anspruch 11, wobei die Bestimmung der Expression von MACC1 eine Hybridisierung, eine PCR, eine "real time" (RT)-PCR, eine Antigen-Antikörper Bindung, einen ELISA, eine optische Proteom-Analyse, eine ein- oder mehrdimensionale Gelelektrophorese, eine massenspektrometrische Analyse, eine Chromatographie, eine Sequenzierung, Methylierungsanalyse, SNP-Bestimmung oder Kombinationen dieser Verfahren umfasst.

13. Verfahren nach einem der Ansprüche 10 bis 12, wobei die tumoröse Erkrankung metastasierender Kolonkrebs ist.

14. Verfahren nach einem der Ansprüche 10 bis 13, wobei die biologische Probe aus einer Tumorbiopsie aus Darm, Leber, Lymphknoten, Lunge, Knochen oder Hirn oder Körperflüssigkeiten abgeleitet ist.

15. Verfahren zur Auffindung von Substanzen, die an MACC1 binden, umfassend
a) in Kontakt bringen einer Zelle, die MACC1 exprimiert, mit einer Kandidaten-Substanz,
b) Nachweisen der Anwesenheit der Kandidaten-Substanz, die an MACC1 bindet, und
c) Bestimmen, ob die Kandidaten-Substanz auch an MACC1 bindet,
wobei MACC1 ein Polypeptid, wie in Anspruch 2 definiert, ist.

16. Verfahren zur Herstellung einer pharmazeutischen Zusammensetzung, umfassend die Schritte des Verfahrens nach Anspruch 15 und Formulieren der in Schritt c) identifizierten Substanz in eine pharmazeutisch akzeptable Form, wobei die Substanz ein Antikörper gegen MACC1 ist.

17. Nukleinsäuresequenz nach Anspruch 1, Polypeptid nach Anspruch 2, Oligonukleotid nach Anspruch 3, Antikörper nach Anspruch 7 oder diagnostische Zusammensetzung nach Anspruch 9 zur Verwendung bei der Diagnose von tumorösen Erkrankungen, wobei die tumoröse Erkrankung Kolonkrebs ist, oder zur Prognose der Wahrscheinlichkeit der Fernmetastasierung von Kolonkrebs.

18. Diagnostischer Kit, umfassend eine diagnostische Zusammensetzung nach Anspruch 9, gegebenenfalls zusammen mit geeigneten Puffern und/oder Gebrauchsanweisungen.

19. Diagnostischer Kit nach Anspruch 18 in Form eines PCR-, insbesondere RT-PCR-, oder ELISA-Kits.

## Claims

1. Nucleic acid sequence coding for the polypeptide of MACC1, selected from the group of:
a) a nucleic acid sequence having the sequence as given in SEQ ID No: 1,
b) nucleic acid sequences derived from said nucleic acid sequence as given in SEQ ID No: 1 as a result of the degenerated genetic code.

2. MACC1 polypeptide having the sequence as given in SEQ ID No: 2, encoded by a nucleic acid sequence according to claim 1.

3. Oligonucleotide, which specifically hybridizes to a nucleic acid sequence according to claim 1, having the nucleic acid sequence of SEQ ID No: 7.

4. Nucleic acid molecule according to claim 1, polypeptide according to claim 2 or oligonucleotide according to claim 3 as a medicament.

5. Vector containing a nucleic acid sequence according to claim 1.

6. Recombinant prokaryotic or non-human eukaryotic host organism containing at least one vector according to claim 5.

7. Polyclonal or monoclonal antibody or antigen-binding fragment thereof, which recognizes a MACC1 polypeptide having SEQ ID No: 2.

8. Pharmaceutical composition comprising a nucleic acid sequence according to claim 1, a polypeptide according to claim 2, an oligonucleotide according to claim 3 or an antibody according to claim 7, optionally in combination with a pharmaceutically acceptable carrier.

9. Diagnostic composition comprising a nucleic acid sequence according to claim 1, a polypeptide according to claim 2, an oligonucleotide according to claim 3 or an antibody according to claim 7.

10. Method for the diagnosis of tumour diseases, comprising the step of determining the expression of MACC 1 in a biological sample from a pathologic tissue or bodily fluids and comparing said expression with the expression of MACC1 in a healthy tissue or bodily fluid, wherein the tumour disease is colon cancer,
wherein MACC1 is a polypeptide as defined in claim 2.

11. Method for the prognosis of the probability of the formation of distant metastases in colon cancer, comprising the step of determining the expression of MACC1 in a biological sample from a pathologic tissue or bodily fluids and comparing said expression with the expression of MACC1 in a healthy tissue or bodily fluid,
wherein MACC1 is a polypeptide as defined in claim 2.

12. Method for the diagnosis of colon cancer according to claim 10 or for the prognosis of the probability of the formation of distant metastases in colon cancer according to claim 11, wherein the determination of said expression of MACC1 comprises a hybridization, a PCR, a real time (RT)-PCR, an antigen-antibody binding, an ELISA, an optical proteome analysis, a one- or multi-dimensional gel electrophoresis, an analysis by mass spectrometry, a chromatography, a sequencing procedure, a methylation analysis, a SNP-determination or combinations of these methods.

13. Method according to any one of claims 10 to 12, wherein said tumour disease is metastasising colon cancer.

14. Method according to any one of claims 10 to 13, wherein said biological sample is derived from a tumour biopsy from the intestine, liver, lymph nodes, lung, bones or brain or from bodily fluids.

15. Method for the identification of compounds which bind to MACC1, the method comprising:
a) contacting a cell expressing MACC1 with a candidate compound,
b) detecting the presence of the candidate compound which binds to MACC1, and
c) determining whether the candidate compound also binds to MACC1,
wherein MACC1 is a polypeptide as defined in claim 2.

16. Method for the preparation of a pharmaceutical composition, comprising the steps of the method according to claim 15 and the formulation of the compound identified in step c) in a pharmaceutically acceptable form,
wherein the compound is an antibody against MACC1.

17. Nucleic acid sequence according to claim 1, polypeptide according to claim 2, oligonucleotide according to claim 3, antibody according to claim 7 or diagnostic composition according to claim 9 for the use in the diagnosis of tumour diseases, wherein the tumour disease is colon cancer, or for the prognosis of the probability of the formation of distant metastases in colon cancer.

18. Diagnostic kit comprising a diagnostic composition according to claim 9, optionally also containing suitable buffers and/or operating instructions.

19. Diagnostic kit according to claim 18 in the form of a PCR kit, in particular a RT-PCR kit, or an ELISA kit.

## Revendications

1. Séquence d'acides nucléiques qui code pour le polypeptide à partir de MACC1, choisie dans le groupe :
a) d'une séquence d'acides nucléiques comprenant la séquence indiquée dans la SEQ ID N° : 1,
b) de séquences d'acides nucléiques qui constituent le résultat dérivé du code génétique dégénéré de la séquence d'acides nucléiques indiquée dans la SEQ ID N° : 1.

2. Polypeptide MACC1 comprenant la séquence d'acides aminés indiquée dans la SEQ ID N ° : 2, codé par une séquence d'acides nucléiques selon la revendication 1.

3. Oligonucléotide qui s'hybride de manière spécifique à une séquence d'acides nucléiques selon la revendication 1 avec la séquence d'acides nucléiques de la SEQ ID N ° : 7.

4. Molécule d'acide nucléique selon la revendication 1, polypeptide selon la revendication 2 ou oligonucléotide selon la revendication 3 servant de produit pharmaceutique.

5. Vecteur contenant une séquence d'acides nucléiques selon la revendication 1.

6. Organisme hôte recombinant procaryote ou eucaryote non humain contenant au moins un vecteur selon la revendication 5.

7. Anticorps polyclonal ou monoclonal ou un fragment liant un antigène de celui-ci, qui reconnaît un polypeptide MACC1 comprenant la SEQ ID N ° : 2.

8. Composition pharmaceutique comprenant une séquence d'acides nucléiques selon la revendication 1, un polypeptide selon la revendication 2, un oligonucléotide selon la revendication 3 ou un anticorps selon la revendication 7, éventuellement de manière conjointe avec un support pharmaceutiquement acceptable.

9. Composition de diagnostic comprenant une séquence d'acides nucléiques selon la revendication 1, un polypeptide selon la revendication 2, un oligonucléotide selon la revendication 3 ou un anticorps selon la revendication 7.

10. Procédé de diagnostic de maladies tumorales comprenant l'étape de la détermination de l'expression de MACC1 dans un échantillon biologique d'un tissu malade ou d'un liquide corporel et la comparaison de l'expression avec l'expression de MACC1 dans un tissu ou un liquide corporel sain, la maladie tumorale étant le cancer du colon,
MACC1 étant un polypeptide tel que défini dans la revendication 2.

11. Procédé de prévision de la probabilité de la formation de métastases à distance du cancer du colon, comprenant l'étape de détermination de l'expression de MACC1 dans un échantillon biologique constitué d'un tissu ou d'un liquide corporel malade et l'étape de comparaison de l'expression avec l'expression de MACC1 dans un tissu ou un liquide corporel sain,
MACC1 étant un polypeptide tel que défini dans la revendication 2.

12. Procédé de diagnostic du cancer du colon selon la revendication 10 ou de prévision de la probabilité d'une formation de métastases à distance du cancer du colon selon la revendication 11, la détermination de l'expression de MACC1 comprenant une hybridation, une PCR, une amplification RT-PCR en « temps réel », une liaison antigène-anticorps, un test ELISA, une analyse optique de protéome, un électrophorèse sur gel mono- ou multi-dimensionnelle, une analyse en spectrométrie de masse, une chromatographie, un séquençage, une analyse par méthylation, une détermination de SNP ou une combinaison de ces procédés.

13. Procédé selon l'une des revendications de 10 à 12, la maladie tumorale étant le cancer du colon métastatique.

14. Procédé selon l'une des revendications de 10 à 13, l'échantillon biologique étant dérivé d'une biopsie de tumeur dans l'intestin, le foie, les ganglions lymphatiques, le poumon, l'os ou le cerveau ou de liquides corporels.

15. Procédé pour retrouver des substances qui se lient à MACC1 comprenant
a) la mise en contact d'une cellule qui exprime MACC1 avec une substance candidate,
b) la preuve de la présence de la substance candidate qui se lie à MACC1, et
c) la détermination si la substance candidate se lie également à MACC1,
MACC1 étant un polypeptide tel que défini dans la revendication 2.

16. Procédé de fabrication d'une composition pharmaceutique comprenant les étapes du procédé selon la revendication 15 et la formulation de la substance identifiée dans l'étape c) sous une forme pharmaceutiquement acceptable, la substance étant un anticorps anti-MACC1.

17. Séquence d'acides nucléiques selon la revendication 1, polypeptide selon la revendication 2, oligonucléotide selon la revendication 3, anticorps selon la revendication 7 ou composition de diagnostic selon la revendication 9 pour une utilisation lors du diagnostic de maladies tumorales, la maladie tumorale étant le cancer du colon, ou pour une prévision de la probabilité de la formation de métastases à distance du cancer du colon.

18. Kit de diagnostic comprenant une composition de diagnostic selon la revendication 9, éventuellement de manière conjointe avec des milieux tampons appropriés et/ou les conseils d'utilisation.

19. Kit de diagnostic selon la revendication 18 sous la forme d'un kit de PCR, en particulier de RT-PCR, ou d'ELISA.


### SEQUENZPROTOKOLL

<110> Charite - Universitätsmedizin Berlin
<120> 7a5/Prognostin und dessen Verwendung für die Tumordiagnostik und Tumortherapie
<130> U30057PCT
<160> 7
<170> Patent In version 3.2
<210> 1
   <211> 2559
   <212> DNA
   <213> Homo sapiens
<400> 1
   atgctaatca ctgaaagaaa acattttcgg tcaggaagaa ttgcacaaag tatgtctgaa     60
   gcaaatttga ttgacatgga agctggaaaa ctctcaaaaa gttgcaatat tacagaatgc    120
   caggacccag acttgcttca caattggccg gatgctttca cccttcgtgg taataatgct    180
   tccaaagttg caaatccatt ctggaatcaa ctgtctgctt ctaacccatt tttggatgac    240
   ataactcaac taagaaataa caggaagaga aataatattt ccatcttaaa ggaagatcct    300
   tttcttttct gtagagaaat agaaaatgga aattcttttg attcctccgg tgatgaactt    360
   gatgtgcatc agttacttag gcagacttcc tcaagaaatt ctggaagatc taaaagtgtt    420
   tcagaacttc tggacatttt agacgacaca gcacatgccc atcagagtat acataactct    480
   gaccagatcc tactacacga cttagagtgg cttaaaaatg atcgggaggc ttataaaatg    540
   gcttggttaa gtcaacgcca gctggcccgc tcctgccttg atttgaatac aattagtcag    600
   agccctggat gggcccagac acaacttgcg gaggtcacca tagcttgcaa agtaaaccat    660
   caaggagggt cagtacaatt acctgaatca gacatcactg ttcatgtgcc ccaaggtcat    720
   gtggctgtgg gagaattcca agaggtgtct ctaagggctt tccttgatcc gccacacatg    780
   cttaaccatg atctttcgtg cactgtgagc ccgttgttgg aaatcatgtt aggcaacctc    840
   aatacaatgg aagccctttt gctggagatg aaaattgggg ctgaagtaag aaaggatcct    900
   ttcagccaag tcatgacaga aatggtgtgt ttacacagct tgggtaaaga aggccctttt    960
   aaagttttaa gcaactgcta catttataaa gacaccatcc aagtcaagct aatcgacttg   1020
   agtcaggtaa tgtatctagt ggttgctgca caagctaaag ctcttccgtc accagctgcc   1080
   accatttggg attatatcca caaaaccacc tcaattggaa tttatggacc caaatatatc   1140
   catcccagtt ttactgttgt tttaacagtt tgtggacaca attatatgcc aggacagctt   1200
   acaatttctg atattaagaa gggtggaaaa aacatatctc cagttgtgtt tcagctctgg   1260
   gggaagcagt catttttact tgacaagcca caagatttaa gtatttctat tttttcctgt   1320
   gatcctgatt ttgaagtaaa gacagaagga gaaaggaaag aaattaaaca aaagcagttg   1380
   gaagcaggtg aagtagttca tcaacaattt ttattttctt tagttgagca cagagagatg   1440
   cacttgtttg atttttgtgt tcaagtggag cctcccaatg gtgaaccagt tgcacagttc   1500
   tctatcacta ctcctgatcc aaccccaaac ctaaaaagac tctcgaatct gccaggctat   1560
   ttgcagaaga aggaggaaat caagtctgct cctttatcac caaaaattct tgttaaatat   1620
   cctacatttc aagataaaac attgaacttt agcaactatg gggtaaccct gaaggcagtg   1680
   ctaagacaaa gcaagattga ttacttcctt gaatatttca aaggggacac aatagctctc   1740
   ctcggggaag gtaaggtaaa agctattggt cagtccaaag tgaaagaatg gtatgtagga   1800
   gtcctcagag gtaagattgg acttgtacac tgcaaaaatg tcaaggtgat ttcaaaggag   1860
   caagtaatgt ttatgtcaga tagtgtcttt acaaccagaa atcttcttga acagattgtc   1920
   ctgcctttaa aaaaattgac ttatatctac tcagttgtat taaccttggt gtcagaaaaa   1980
   gtttatgatt ggaaagtttt agctgatgtc ctgggttact cacatctgtc cctggaagat   2040
   tttgatcaaa ttcaagcaga caaagaatca gagaaagttt cttatgttat aaagaagtta   2100
   aaggaagatt gccacacaga gagaaataca aggaagtttc tgtatgaact tattgtggct   2160
   cttctgaaaa tggattgcca agagttagtc gcacgtctca tccaagaagc tgctgttctg   2220
   acttcagctg tcaagcttgg aaaaggctgg agggaactag ctgaaaagtt agtacgactc   2280
   acaaagcaac aaatggaggc atatgaaatt cctcatcgag gaaacactgg agatgttgct   2340
   gttgagatga tgtggaaacc tgcctatgat tttctgtata cctggagtgc tcactatgga   2400
   aataactaca gagatgtgtt acaagacctt cagtcagctt tggacagaat gaaaaaccct   2460
   gtgactaaac actggagaga attaactgga gttttaatac tagtaaattc tttggaggtt   2520
   ttgagagtaa ctgcattctc cacttctgag gaagtatag                          2559
<210> 2
   <211> 852
   <212> PRT
   <213> Homo sapiens
<400> 2
   Met Leu Ile Thr Glu Arg Lys His Phe Arg Ser Gly Arg Ile Ala Gln
   1               5                   10                  15
   Ser Met Ser Glu Ala Asn Leu Ile Asp Met Glu Ala Gly Lys Leu Ser
               20                  25                  30
   Lys Ser Cys Asn Ile Thr Glu Cys Gln Asp Pro Asp Leu Leu His Asn
           35                  40                  45
   Trp Pro Asp Ala Phe Thr Leu Arg Gly Asn Asn Ala Ser Lys Val Ala
           50                  55                  60
   Asn Pro Phe Trp Asn Gln Leu Ser Ala Ser Asn Pro Phe Leu Asp Asp
   65                  70                  75                  80
   Ile Thr Gln Leu Arg Asn Asn Arg Lys Arg Asn Asn Ile Ser Ile Leu
                   85                  90                  95
   Lys Glu Asp Pro Phe Leu Phe Cys Arg Glu Ile Glu Asn Gly Asn Ser
               100                 105                 110
   Phe Asp Ser Ser Gly Asp Glu Leu Asp Val His Gln Leu Leu Arg Gln
           115                 120                 125
   Thr Ser Ser Arg Asn Ser Gly Arg Ser Lys Ser Val Ser Glu Leu Leu
       130                 135                 140
   Asp Ile Leu Asp Asp Thr Ala His Ala His Gln Ser Ile His Asn Ser
   145                 150                 155                 160
   Asp Gln Ile Leu Leu His Asp Leu Glu Trp Leu Lys Asn Asp Arg Glu
                   165                 170                 175
   Ala Tyr Lys Met Ala Trp Leu Ser Gln Arg Gln Leu Ala Arg Ser Cys
               180                 185                 190
   Leu Asp Leu Asn Thr Ile Ser Gln Ser Pro Gly Trp Ala Gln Thr Gln
           195                 200                 205
   Leu Ala Glu Val Thr Ile Ala Cys Lys Val Asn His Gln Gly Gly Ser
       210                 215                 220
   Val Gln Leu Pro Glu Ser Asp Ile Thr Val His Val Pro Gln Gly His
   225                 230                 235                 240
   Val Ala Val Gly Glu Phe Gln Glu Val Ser Leu Arg Ala Phe Leu Asp
                   245                 250                 255
   Pro Pro His Met Leu Asn His Asp Leu Ser Cys Thr Val Ser Pro Leu
               260                 265                 270
   Leu Glu Ile Met Leu Gly Asn Leu Asn Thr Met Glu Ala Leu Leu Leu
           275                 280                 285
   Glu Met Lys Ile Gly Ala Glu Val Arg Lys Asp Pro Phe Ser Gln Val
       290                 295                 300
   Met Thr Glu Met Val Cys Leu His Ser Leu Gly Lys Glu Gly Pro Phe
   305                 310                 315                 320
   Lys Val Leu Ser Asn Cys Tyr Ile Tyr Lys Asp Thr Ile Gln Val Lys
                   325                 330                 335
   Leu Ile Asp Leu Ser Gln Val Met Tyr Leu Val Val Ala Ala Gln Ala
               340                 345                 350
   Lys Ala Leu Pro Ser Pro Ala Ala Thr Ile Trp Asp Tyr Ile His Lys
           355                 360                 365
   Thr Thr Ser Ile Gly Ile Tyr Gly Pro Lys Tyr Ile His Pro Ser Phe
       370                 375                 380
   Thr Val Val Leu Thr Val Cys Gly His Asn Tyr Met Pro Gly Gln Leu
   385                 390                 395                 400
   Thr Ile Ser Asp Ile Lys Lys Gly Gly Lys Asn Ile Ser Pro Val Val
                   405                 410                 415
   Phe Gln Leu Trp Gly Lys Gln Ser Phe Leu Leu Asp Lys Pro Gln Asp
               420                 425                 430
   Leu Ser Ile Ser Ile Phe Ser Cys Asp Pro Asp Phe Glu Val Lys Thr
           435                 440                 445
   Glu Gly Glu Arg Lys Glu Ile Lys Gln Lys Gln Leu Glu Ala Gly Glu
       450                 455                 460
   Val Val His Gln Gln Phe Leu Phe Ser Leu Val Glu His Arg Glu Met
   465                 470                 475                 480
   His Leu Phe Asp Phe Cys Val Gln Val Glu Pro Pro Asn Gly Glu Pro
                   485                 490                 495
   Val Ala Gln Phe Ser Ile Thr Thr Pro Asp Pro Thr Pro Asn Leu Lys
               500                 505                 510
   Arg Leu Ser Asn Leu Pro Gly Tyr Leu Gln Lys Lys Glu Glu Ile Lys
           515                 520                 525
   Ser Ala Pro Leu Ser Pro Lys Ile Leu Val Lys Tyr Pro Thr Phe Gln
       530                 535                 540
   Asp Lys Thr Leu Asn Phe Ser Asn Tyr Gly Val Thr Leu Lys Ala Val
   545                 550                 555                 560
   Leu Arg Gln Ser Lys Ile Asp Tyr Phe Leu Glu Tyr Phe Lys Gly Asp
   565 570 575
   Thr Ile Ala Leu Leu Gly Glu Gly Lys Val Lys Ala Ile Gly Gln Ser
   580 585 590
   Lys Val Lys Glu Trp Tyr Val Gly Val Leu Arg Gly Lys Ile Gly Leu
   595 600 605
   Val His Cys Lys Asn Val Lys Val Ile Ser Lys Glu Gln Val Met Phe
   610 615 620
   Met Ser Asp Ser Val Phe Thr Thr Arg Asn Leu Leu Glu Gln Ile Val
   625 630 635 640
   Leu Pro Leu Lys Lys Leu Thr Tyr Ile Tyr Ser Val Val Leu Thr Leu
   645 650 655
   Val Ser Glu Lys Val Tyr Asp Trp Lys Val Leu Ala Asp Val Leu Gly
   660 665 670
   Tyr Ser His Leu Ser Leu Glu Asp Phe Asp Gln Ile Gln Ala Asp Lys
   675 680 685
   Glu Ser Glu Lys Val Ser Tyr Val Ile Lys Lys Leu Lys Glu Asp Cys
   690 695 700
   His Thr Glu Arg Asn Thr Arg Lys Phe Leu Tyr Glu Leu Ile Val Ala
   705 710 715 720
   Leu Leu Lys Met Asp Cys Gln Glu Leu Val Ala Arg Leu Ile Gln Glu
   725 730 735
   Ala Ala Val Leu Thr Ser Ala Val Lys Leu Gly Lys Gly Trp Arg Glu
   740 745 750
   Leu Ala Glu Lys Leu Val Arg Leu Thr Lys Gln Gln Met Glu Ala Tyr
   755 760 765
   Glu Ile Pro His Arg Gly Asn Thr Gly Asp Val Ala Val Glu Met Met
   770 775 780
   Trp Lys Pro Ala Tyr Asp Phe Leu Tyr Thr Trp Ser Ala His Tyr Gly
   785 790 795 800
   Asn Asn Tyr Arg Asp Val Leu Gln Asp Leu Gln Ser Ala Leu Asp Arg
   805 810 815
   Met Lys Asn Pro Val Thr Lys His Trp Arg Glu Leu Thr Gly Val Leu
               820                 825                 830
   Ile Leu Val Asn Ser Leu Glu Val Leu Arg Val Thr Ala Phe Ser Thr
           835                 840                 845
   Ser Glu Glu Val
       850
<210> 3
   <211> 21
   <212> DNA
   <213> Homo sapiens
<400> 3
   ttcttttgat tcctccggtg a 21
<210> 4
   <211> 20
   <212> DNA
   <213> Homo sapiens
<400> 4
   actctgatgg gcatgtgctg 20
<210> 5
   <211> 32
   <212> DNA
   <213> Homo sapiens
<400> 5
   gcagacttcc tcaagaaatt ctggaagatc ta 32
<210> 6
   <211> 32
   <212> DNA
   <213> Homo sapiens
<400> 6
   agtgtttcag aacttctgga cattttagac ga 32
<210> 7
   <211> 21
   <212> DNA
   <213> Homo sapiens
<400> 7
   aagcttggaa aaggctggag g 21